# EUROPEAN PATENT SPECIFICATION

(11) **EP 1 343 879 B1**
(45) Date of publication and mention of the grant of the patent: **08.01.2014**
(21) Application number: 00977013.2
(22) Date of filing: 07.11.2000
(51) Int. Cl.: C12N 9/00, C07K 14/47

(54) **METHODS FOR ISOLATING NOVEL ANTIMICROBIAL AGENTS FROM HYPERMUTABLE MAMMALIAN CELLS**
VERFAHREN ZUR ISOLIERUNG NEUER ANTIMIKROBIELLER AGENTIEN AUS HYPERMUTIERBAREN SÄUGERZELLEN
PROCEDES POUR ISOLER DE NOUVEAUX AGENTS ANTIMICROBIENS A PARTIR DE CELLULES MAMMIFERES HYPERMUTABLES

(43) Date of publication of application: 17.09.2003
(73) Proprietor: Morphotek, Inc., Exton, PA 19341 (US)
(72) Inventor: GRASSO, Luigi, Bryn Mawr, PA 19010 (US); NICOLAIDES, Nicholas, C., Glenn Mills, PA 19342 (US); SASS, Philip, M., Audubon, PA 19403 (US)
(74) Representative: WP Thompson
(86) International application number: PCT/US2000/030587
(87) International publication number: WO 2002/038750

(56) References cited:
- CA-A1- 2 240 609
- US-A- 6 146 894
- PAPADOPOULOS N ET AL: "MUTATION OF MUTL HOMOLOG IN HEREDITARY COLON CANCER" SCIENCE, AMERICAN ASSOCIATION FOR THE ADVANCEMENT OF SCIENCE,, US, vol. 263, 18 March 1994 (1994-03-18), pages 1625-1629, XP000608347 ISSN: 0036-8075
- NICOLAIDES N C ET AL: "Mutations of two PMS homologues in heriditary nonpolyposis colon cancer" NATURE, NATURE PUBLISHING GROUP, LONDON, GB, no. 371, 1 September 1994 (1994-09-01), pages 75-80, XP002079185 ISSN: 0028-0836
- WINTER D B ET AL: "Altered spectra of hypermutation in antibodies from mice deficient for the DNA mismatch repair protein PMS2" PROCEEDINGS OF THE NATIONAL ACADEMY OF SCIENCES OF USA, NATIONAL ACADEMY OF SCIENCE, WASHINGTON, DC, US, vol. 95, no. 12, 9 June 1998 (1998-06-09), pages 6953-6958, XP002321857 ISSN: 0027-8424
- WIESENDANGER MARGRIT ET AL: "Somatic hypermutation, transcription, and DNA mismatch repair" CELL, CELL PRESS, CAMBRIDGE, NA, US, vol. 94, no. 4, 21 August 1998 (1998-08-21), pages 415-418, XP002333265 ISSN: 0092-8674
- REYNAUD C-A ET AL: "Mismatch repair and immunoglobulin gene hypermutation: did we learn something?" IMMUNOLOGY TODAY, ELSEVIER PUBLICATIONS, CAMBRIDGE, GB, vol. 20, no. 11, November 1999 (1999-11), pages 522-527, XP004183761 ISSN: 0167-5699
- NICOLAIDES ET AL.: 'A naturally occurring hPMS2 mutation can confer a dominant negative mutator phenotype' MOLECULAR AND CELLULAR BIOLOGY vol. 18, no. 3, March 1998, pages 1635 - 1641, XP002937919
- MA ET AL.: 'Dominant negative expression of hPMS2 creates isogenic mismatch repair deficient human colon cancer cell lines' PROCEEDINGS OF THE AMERICAN ASSOCIATION FOR CANCER RESEARCH vol. 39, March 1998, page 460, ABSTRACT #3130, XP002937920
- LIU ET AL.: 'Analysis of mismatch repair genes in hereditary non-polyposis colorectal cancer patients' NATURE MEDICINE vol. 2, no. 2, February 1996, pages 169 - 174, XP002937921

## Description

### FIELD OF THE INVENTION

The present invention is related to the area of antimicrobial agents and cellular production of those agents. In particular, it is related to the field of identification of novel antimicrobial agents by placing mammalian cells under selection in the presence of the microbe.

### BACKGROUND OF THE INVENTION

For as long as man has shared the planet with microorganisms there have been widespread outbreaks of infectious disease and subsequent widespread mortality associated with it. Although microorganisms and man frequently share a symbiotic relationship, microorganisms can, under some conditions, lead to sickness and death. The discovery, wide use and dissemination of antibiotics to treat microbial infection in both human and animal populations over the last one hundred or so years has done much to control, and in some instances, eradicate some microbes and associated infectious disease. However, microbes have a strong propensity to evolve and alter their genetic makeup when confronted with toxic substances that place them under life and death selective pressures. Therefore, emerging infectious diseases currently pose an important public health problem in both developed as well as developing countries. Not only have microbes evolved to evade and defeat current antibiotic therapeutics, but also there are novel and previously unrecognized and/or characterized bacterial, fungal, viral, and parasitic diseases that have emerged within the past two decades. Sass, Curr. Opin. in Drug Discov. & Develop. 2000, 3(5):646-654.

Since the accidental discovery of a penicillin-producing mold by Fleming there has been steady progress in synthesizing, isolating and characterizing new and more effective beta-lactam antibiotics. In addition to the great success of the beta-lactam family of antibiotics, the newer fluoroquinolones have a broad-spectrum of bactericidal activity as well as excellent oral bio-availability, tissue penetration and favorable safety and tolerability profiles. King et al., Am. Fam. Physician, 2000, 61, 2741-2748. A newly devised four-generation classification of the quinolone drugs accounts for the expanded antimicrobial spectrum of the more recently introduced fluoroquinolones and their clinical indications. The so-called first generation drugs, which include nalidixic acid, are capable of achieving minimal serum levels. The second-generation quinolones, such as ciprofloxacin, have an increased gram-negative and systemic activity. The third-generation drugs comprise pharmaceuticals such as levofloxacin and are have significant and expanded action against gram-positive bacteria and atypical pathogens. Finally, the fourth-generation quinolone drugs, which, to date, only includes trovofloxacin, are highly active against anaerobes in addition to the activity described for the third-generation drugs. Furthermore, the quinolone class of anti-microbial drugs can be divided based on their pharmacokinetic properties and bioavailability.

Mammalian epithelial surfaces are remarkable for their ability to provide critical physiologic functions in the face of frequent microbial challenges. The fact that these mucosal surfaces remain infection-free in the normal host suggests that highly effective mechanisms of host defense have evolved to protect these environmentally exposed tissues. Throughout the animal and plant kingdoms, endogenous genetically encoded antimicrobial peptides have been shown to be key elements in the response to epithelial compromise and microbial invasion. Zasloff, Curr. Opin. Immunol., 1992, 4, 3-7; and Bevins, Ciba Found. Symp., 1994, 186, 250-69. In mammals, a variety of such peptides have been identified, including the well-characterized defensins and cathelicidins and others (andropin, magainin, tracheal antimicrobial peptide, and PR-39; see Bevins, Ciba Found. Symp., 1994, 186, 250-69 and references therein). A major source of these host defense molecules is circulating phagocytic leukocytes. However, more recently, it has been shown that resident epithelial cells of the skin and respiratory, alimentary, and genitourinary tracts also synthesize and release antimicrobial peptides. Both *in vitro* and *in vivo* data support the hypothesis that these molecules are important contributors to intrinsic mucosal immunity. Alterations in their level of expression or biologic activity can predispose the organism to microbial infection. Huttner et al., Pediatr. Res., 1999, 45, 785-94.

Across the evolutionary scale species from insects to mammals to plants defend themselves against invading pathogenic microorganisms by utilizing cationic antimicrobial peptides that rapidly kill microbes without exerting toxicity to the host. Physicochemical peptide-lipid interactions provide attractive mechanisms for innate immunity as discussed below. Many of these peptides form cationic amphipathic secondary structures, typically alpha-helices and beta-sheets, which can selectively interact with anionic bacterial membranes via electrostatic interactions. Rapid, peptide-induced membrane permeabilization and subsequent cellular lysis is the result. Matsuzaki, Biochim. Biophys. Acta, 1999, 1462, 1-10.

The primary structures of a large number of these host-defense peptides have been determined. While there is no primary structure homology, the peptides are characterized by a preponderance of cationic and hydrophobic amino acids. The secondary structures of many of the host-defense peptides have been determined by a variety of techniques. Sitaram et al., Biochim, Biophys. Acta, 1999, 1462, 29-54. The acyclic peptides tend to adopt helical conformation, especially in media of low dielectric constant, whereas peptides with more than one disulfide bridge adopt beta-structures.

As described above, one reason for the rise in microbial drug resistance to the first line antimicrobial therapies in standard use today is the inappropriate and over-use of prescription antibiotics. Although bacteria are the most common organisms to develop drug-resistance, there are numerous examples of demonstrated resistance in fungi, viruses, and parasites. The development of a resistant phenotype is a complex phenomenon that involves an interaction of the microorganism, the environment, and the patient, separately as well as in combination. Sitaram et al., Biochim. Biophys. Acta, 1999, 1462, 29-54. The microorganism in question may develop resistance while under antibiotic selection or it may be a characteristic of the microbe prior to exposure to a given agent. There are a number of mechanisms of resistance to antibiotics that have been described, including genes that encode antibiotic resistance enzymes that are harbored on extrachromosomal plasmids as well as DNA elements (e.g. transposable elements) that can reside either extra-chromosomally or within the host genome.

Due to the ability of microorganisms to acquire the ability to develop resistance to antibiotics there is a need to continually develop novel antibiotics. Traditional methods to develop novel antibiotics have included medicinal chemistry approaches to modify existing antibiotics (Kang et al., Bioorg. Med. Chem. Lett., 2000, 10, 95-99) as well as isolation of antibiotics from new organisms (Alderson et al., Res. Microbiol., 1993, 144, 665-72). Each of these methods, however, has limitations. The traditional medicinal chemistry approach entails modification of an existing molecule to impart a more effective activity. The chemist makes a "best guess" as to which parts of the molecule to alter, must then devise a synthetic strategy, synthesize the molecule, and then have it tested. This approach is laborious, requires large numbers of medicinal chemists and frequently results in a molecule that is lower in activity than the original antibiotic. The second approach, isolation of novel antimicrobial agents, requires screening large numbers of diverse organisms for novel antimicrobial activity. Then, the activity must be isolated from the microorganism. This is not a small task, and frequently takes many years of hard work to isolate the active molecule. Even after the molecule is identified, it may not be possible for medicinal chemists to effectively devise a synthetic strategy due to the complexity of the molecule. Furthermore, the synthetic strategy must allow for a cost-effective synthesis.

CA2240609 discloses dominant negative alleles of human mismatch repair genes, that can be used to generate hypermutable cells and organisms. By introducing these genes into cells and transgenic animals, new cell lines and animal varieties with novel and useful properties were prepared more efficiently than relying on the natural rate of mutation. Papadopoulos et al. ("Mutation of mutL homolog in hereditary colon cancer ", Science, American Association for the Advancement of Science, US, 263, 18 March 1994, pages 1625-1629*)* discloses that some cases of hereditary colorectal cancer are due to alterations in a mutS-related mismatch repair gene. The authors carried out a search of a large database of expressed sequence tags derived from random complimentary DNA clones, which revealed three additional human mismatch repair genes, all related to the bacterial mutL gene. Nicolaides et al. ("Mutations of two PMS homologues in hereditary nonpolyposis colon cancer", Nature, London, GB, 371, 1 September 1994, pages 75-80*)* discloses the nucleotide sequence, chromosome localization and mutational analysis of hPMS1 and hPMS2, two additional homologues of the prokaryotic mutL gene, both of which were found to be mutated in the germ line of colorectal cancer patients. Winter et al. ("Altered spectra of hypermutation in antibodies from mice deficient for the DNA mismatch repair protein PMS2 ", Proceedings of the National Academy of Sciences of USA, Washington, DC, US, 95 (12), 9 June 1998, pages 6953-6958*)* studied hypermutation in mice deficient for either the DNA nucleotide excision repair gene *Xpa* or the mismatch repair gene *Pms2.* High levels of mutation were found in variable genes from XPA-deficient and PMS2-deficient mice, indicating that neither nucleotide excision repair nor mismatch repair pathways generate hypermutation. The authors also confirm that tandem mispairs were repaired by wild-type cells but not by *Pms2*^{*-*/*-*} human or murine cells.

Therefore, a method that would allow for creation of more effective antibiotics from existing molecules or allow rapid isolation of novel antimicrobial agents is needed to combat the ever-growing list of antibiotic resistant organisms. The present invention described herein is directed to the use of random genetic mutation of a cell to produce novel antibiotics by blocking the endogenous mismatch repair activity of a host cell. The cell can be a mammalian cell that produces an antimicrobial agent naturally, or a cell that is placed under selective pressure to obtain a novel antimicrobial molecule that attacks a specific microbe. Moreover, the invention describes methods for obtaining enhanced antimicrobial activity of a cell line that produces an antimicrobial activity due to recombinant expression or as part of the innate capacity of the cell to harbor such activity.

In addition, the generation of genetically altered host cells that are capable of secreting an antimicrobial activity, which can be protein or non-protein based, will be valuable reagents for manufacturing the entity for clinical studies. An embodiment of the invention described herein is directed to the creation of genetically altered host cells with novel and/or increased antimicrobial production that are generated by a method that interferes with the highly ubiquitous and phylogenetically conserved process of mismatch repair.

The present invention facilitates the generation of novel antimicrobial agents and the production of cell lines that express elevated levels of antimicrobial activity. Advantages of the present invention are further described in the examples and figures described herein.

### SUMMARY OF THE INVENTION

One aspect of the invention provides a method for obtaining a mammalian cell that is resistant to a selected microbe, comprising: growing a culture of mammalian cells wherein said cells comprise *hPMS2-134*; exposing said cells to said selected microbe; and selecting said mammalian cell that is resistant to said selected microbe.

A further aspect provides a method for obtaining a cell that comprises a mutation in a gene encoding microbial resistance, comprising: growing a culture of mammalian cells comprising said gene encoding said microbial resistance and *hPMS2-134*; selecting a cell having said microbial resistance; and determining whether said gene comprises a mutation.

The ability of the cell to correct natural defects that occur in the DNA during the process of DNA replication is therefore altered.
Interference with this process, called mismatch repair, leads to genetically dissimilar sibling cells. These genetically dissimilar cells contain mutations, ranging from one mutation/genome to two or more mutations/genome, offer a rich population of cells from which to select for specific output traits, such as the novel ability to resist microbial insult. The genetically altered cell generated by manipulation of the mismatch repair process is then incubated with a microbe that is normally toxic to cells. Most of the cells will rapidly lose viability and die; however, a subset of resistant cells will have the capacity to resist the microbial insult. These cells express a molecule, protein or non-protein in structure, that imbues an antimicrobial activity to the newly selected mammalian clones. These newly created cells can be expanded *in vitro* and the new molecule isolated and characterized by standard methods that are well described it the art. The novel molecule(s) are then tested for their ability to kill or inhibit the growth of the microbe by standard microbial assays that are well described in the art. Finally, the novel cell line generated serves as an additional resource for large-scale production of the novel antimicrobial agent for use in clinical studies. The processes described herein are applicable to any mammalian cell and any microbe for which an antibiotic agent is sought.

In one embodiment of the invention, the step of selecting for microbial resistance comprises isolating and testing culture medium from said cell.

In another embodiment of the invention, the cell is selected for resistance to a bacterium. Alternatively, the cell is selected for resistance to a fungus.

A further embodiment of the present invention provides a method as disclosed above, wherein said step of selecting a cell having said microbial resistance comprises isolating and testing culture medium from said cell.

In another embodiment of the invention, the step of determining whether the gene comprises a mutation comprises analysing a nucleotide sequence of said gene. Alternatively, mRNA transcribed from said gene can be analysed. In a related embodiment, a protein encoded by said gene can be analysed. Alternatively, a phenotypic trait determined by said gene can be analysed.

### BRIEF DESCRIPTION OF THE DRAWINGS

**Figure 1** is a representative *in situ* β-galactosidase staining of TK-hPMS2-134 or TKvect cells to measure for cells containing genetically altered β-galactosidase genes; arrows indicate Blue (β-galactosidase positive) cells.
**Figure 2** is a schematic representation of sequence of alterations of the β-galactosidase gene produced by expression of TK-hPMS2-134 host cells in TK cells.
**Figures 3A, 3B** and **3C** show a representative immunoprecipitation of *in vitro* translated hPMS2 and hMLH1 proteins.
**Figure 4** shows representative complementation of MMR activity in transduced SH cells.
**Figure 5** is a representative photograph of Syrian hamster TK-ts13 cells transfected with a eukaryotic expression vector that produces a novel anti-microbial polypeptide.
**Figure 6** is a representative graph showing TK-hPMS-134 transfected TK cells can suppress the growth of bacteria *in vitro.*

Mismatched repair process is described in several references. Baker et. al., Cell, 1995, 82, 309-319; Bronner et. al., Nature, 1994, 368, 258-261; de Wind et. al., Cell, 1995, 82, 321-330; and Drummond et. al., Science, 1995, 268, 1909-1912. Dominant negative alleles of such genes, when introduced into cells or transgenic animals, increase the rate of spontaneous mutations by reducing the effectiveness of DNA repair and thereby render the cells or animals hypermutable. Hypermutable cells or animals can then be utilized to develop new mutations in a gene of interest or in a gene whose function has not been previously described. Blocking mismatch repair in cells such as, for example, mammalian cells or mammalian cells transfected with genes encoding for specific antimicrobial peptides or non-peptide based antimicrobials, can enhance the rate of mutation within these cells leading to clones that have novel or enhanced antimicrobial activity or production and/or cells that contain genetically altered antimicrobials with enhanced biochemical activity against a range of opportunistic microbes.

The process of mismatch repair, also called mismatch proofreading, is carried out by protein complexes in cells ranging from bacteria to mammalian cells. Modrich, Science, 1994, 266, 1959-1960. A mismatch repair gene is a gene that encodes for one of the proteins of such a mismatch repair complex. Baker et. al., Cell, 1995, 82, 309-319; Bronner et. al., Nature, 1994, 368, 258-261; de Wind et. al., Cell, 1995, 82, 321-330; Drummond et. al., Science, 1995, 268, 1909-1912; and Modrich, Science, 1994, 266, 1959-1960. Although not wanting to be bound by any particular theory of mechanism of action, a mismatch repair complex is believed to detect distortions of the DNA helix resulting from non-complementary pairing of nucleotide bases. The non-complementary base on the newer DNA strand is excised, and the excised base is replaced with the appropriate base that is complementary to the older DNA strand. In this way, cells eliminate many mutations, which occur as a result of mistakes in DNA replication.

Dominant negative alleles cause a mismatch repair defective phenotype even in the presence of a wild-type allele in the same cell. An example of a dominant negative allele of a mismatch repair gene is the human gene *hP MS2-134,* which carries a truncation mutation at codon 134. Nicolaides et. al., Mol. Cell. Biol., 1998, 18, 1635-1641. The mutation causes the product of this gene to abnormally terminate at the position of the 134th amino acid, resulting in a shortened polypeptide containing the N-terminal 133 amino acids. Such a mutation causes an increase in the rate of mutations that accumulate in cells after DNA replication. Expression of a dominant negative allele of a mismatch repair gene results in impairment of mismatch repair activity, even in the presence of the wild-type allele. Any allele, which produces such effect, can be used in this invention.

Dominant negative alleles of a mismatch repair gene can be obtained from the cells of humans, animals, yeast, bacteria, or other organisms. Prolla et. al., Science, 1994, 264, 1091-1093; Strand et. al., Nature, 1993, 365, 274-276; and Su et al., J. Biol. Chem., 1988, 263, 6829-6835. Screening cells for defective mismatch repair activity can identity such alleles. Cells from animals or humans with cancer can be screened for defective mismatch repair. Cells from colon cancer patients may be particularly useful. Parsons et. al., Cell, 1993, 75, 1227-1236; and Papadopoulos et. al., Science, 1993, 263, 1625-1629. Genomic DNA, cDNA, or mRNA from any cell encoding a mismatch repair protein can be analyzed for variations from the wild type sequence. Perucho, Biol. Chem., 1996, 377, 675-684. Dominant negative alleles of a mismatch repair gene can also be created artificially, for example, by producing variants of the *hP MS2-134* allele or other mismatch repair genes. Various techniques of site-directed mutagenesis can be used. The suitability of such alleles, whether natural or artificial, for use in generating hypermutable cells or animals can be evaluated by testing the mismatch repair activity caused by the allele in the presence of one or more wild-type alleles, to determine if it is a dominant negative allele.

A cell or an animal into which a dominant negative allele of a mismatch repair gene has been introduced will become hypermutable. This means that the spontaneous mutation rate of such cells or animals is elevated compared to cells or animals without such alleles. The degree of elevation of the spontaneous mutation rate can be at least 2-fold, 5-fold, 10-fold, 20-fold, 50-fold, 100-fold, 200-fold, 500-fold, or 1000-fold that of the normal cell or animal.

According to one aspect of the invention, a method for obtaining a mammalian cell that is resistant to a selected microbe, comprising: growing a culture of mammalian cells wherein said cells comprise *hP MS2-134*; exposing said cells to said selected microbe; and selecting said mammalian cell that is resistant to said selected microbe is provided. The dominant negative allele *hPMS2-134* can be naturally occurring or made in the laboratory.

A polynucleotide including the dominant negative allele can be cloned into an expression vector containing a constitutively active promoter segment (such as but not limited to CMV, SV40, Elongation Factor or LTR sequences) or to inducible promoter sequences such as the steroid inducible pIND vector (In vitrogen), where the expression of the dominant negative mismatch repair gene can be regulated. The polynucleotide can be introduced into the cell by transfection.

Transfection is any process whereby a polynucleotide is introduced into a cell. The process of transfection can be carried out in a living animal, *e.g*., using a vector for gene therapy, or it can be carried out *in vitro*, *e.g.,* using a suspension of one or more isolated cells in culture. The cell can be any type of eukaryotic cell, including, for example, cells isolated from humans or other primates, mammals or other vertebrates, invertebrates, and single celled organisms such as protozoa, yeast, or bacteria.

In general, transfection will be carried out using a suspension of cells, or a single cell, but other methods can also be applied as long as a sufficient fraction of the treated cells or tissue incorporates the polynucleotide so as to allow transfected cells to be grown and utilized. The protein product of the polynucleotide may be transiently or stably expressed in the cell. Techniques for transfection are well known and available techniques for introducing polynucleotides include but are not limited to electroporation, transduction, cell fusion, the use of calcium chloride, and packaging of the polynucleotide together with lipid for fusion with the cells of interest. Once a cell has been transfected with the mismatch repair gene, the cell can be grown and reproduced in culture. If the transfection is stable, such that the gene is expressed at a consistent level for many cell generations, then a cell line results.

An isolated cell is a cell obtained from a tissue of humans or animals by mechanically separating out individual cells and transferring them to a suitable cell culture medium, either with or without pretreatment of the tissue with enzymes, *e.g*., collagenase or trypsin. Such isolated cells are typically cultured in the absence of other types of cells. Cells selected for the introduction of a dominant negative allele of a mismatch repair gene may be derived from a eukaryotic organism in the form of a primary cell culture or an immortalized cell line, or may be derived from suspensions of single-celled organisms.

The imethod described herein is useful for creating microbial-resistant mammalian cells that secrete new antimicrobial biochemical agents, either protein or non-protein in nature. Furthermore, the method can be applied to cell lines that express known antimicrobial agents as a means to enhance the biochemical activity of the antimicrobial agent

Once a transfected cell line has been produced, it can be used to generate new mutations in one or more gene(s) of interest or in genes that have not been previously described. A gene of interest can be any gene naturally possessed by the cell line or introduced into the cell line by standard methods known in the art. An advantage of using transfected cells or to induce mutation(s) in a gene or genes of interest that encode antimicrobial activity is that the cell need not be exposed to mutagenic chemicals or radiation, which may have secondary harmful effects, both on the object of the exposure and on the workers. Furthermore, it has been demonstrate that chemical and physical mutagens are base pair specific in the way they alter the structure of DNA; the invention described herein results in mutations that are not dependent upon the specific nucleotide or a specific string of nucleotides and is a truly random genetic approach. Therefore, use of the present invention to obtain mutations in novel or known antimicrobial genes will be much more efficient and have a higher likelihood of success in contrast to conventional mutagenesis with chemical or irradiation. Once a new antimicrobial trait is identified in a sibling cell, the dominant negative allele can be removed from the cell by a variety of standard methods known in the art. For example, the gene can be directly knocked out the allele by technologies used by those skilled in the art or use of a inducible expression system; the dominant-negative allele is driven by a standard promoter that is regulated by inclusion of an inducer, withdrawal of the inducer results in attenuation of the expression of the dominant negative mismatch repair mutant and a normal DNA repair process will ensue.

New antimicrobial agents are selected from cells that have been exposed to the dominant negative mismatch repair process followed by incubating the mutant cells in the presence of the microbe for which an novel antimicrobial agent is sought The novel antimicrobial agent is purified by standard methods known to those skilled in the art and characterized. The antimicrobial agents are re-screened to determine the specific activity of the novel antimicrobial as well as tested against a broad range of microbes to determine spectrum of activity. The gene(s) that encode the novel antimicrobial are isolated by standard well known methods to those in the art. The mutations can be detected by analyzing for alterations in the genotype of the cells by examining the sequence of genomic DNA, cDNA, messenger RNA, or amino acids associated with the gene of interest. A mutant polypeptide can be detected by identifying alterations in electrophoretic mobility, spectroscopic properties, or other physical or structural characteristics of a protein encoded by a mutant gene when the cell that has undergone alteration encodes a known antimicrobial that is altered by the means described in the current invention to obtain a more efficacious antimicrobial.

Examples of mismatch repair proteins and nucleic acid sequences include the following:
PMS2 (mouse) (SEQ ID NO:7)
PMS2 (mouse cDNA) (SEQ ID NO:8)
PMS2 (human) (SEQ ID NO:9)
PMS2 (human cDNA) (SEQ ID NO: 10)
PMS1 (human) (SEQ ID NO:11)
PMS1 (human) (SEQ ID NO:12)
MSH2 (human) (SEQ ID NO:13)
MSH2 (human cDNA) (SEQ ID NO:14)
MLH1 (human) (SEQ ID NO:15)
MLH1 (human) (SEQ ID NO:16)
hPMS2-134 (human) (SEQ ID NO:17)
hPMS2-134 (human cDNA) (SEQ ID NO:18)

The above disclosure generally describes the present invention. A more complete understanding can be obtained by reference to the following specific examples, which are provided herein for purposes of illustration only, and are not intended to limit the scope of the invention.

### EXAMPLES

### Example 1: hPMS2-134 Encodes a Dominant Negative Mismatch Repair Protein

A profound defect in MMR was found in the normal cells of two HNPCC patients. That this defect was operative *in vivo* was demonstrated by the widespread presence of microsatellite instability in non-neoplastic cells of such patients. One of the two patients had a germ-line truncating mutation of the *hP MS2* gene at codon 134 (the *hP MS2-134* mutation), while the other patient had a small germ-line deletion within the *hMLH1* gene. Leach et al., Cell, 1993, 75, 1215-1225. These data contradicted the two-hit model generally believed to explain the biochemical and biological features of HNPCC patients. The basis for this MMR deficiency in the normal cells of these patients was unclear, and several potential explanations were offered. For example, it was possible that the second allele of the relevant MMR gene was inactivated in the germ-line of these patients through an undiscovered mechanism, or that unknown mutations of other genes involved in the MMR process were present that cooperated with the known germ-line mutation. It is clear from knock-out experiments in mice that MMR-deficiency is compatible with normal growth and development, supporting these possibilities. Edelmann et al., Cell, 1996, 85, 1125-1134. Alternatively, it was possible that the mutant alleles exerted a dominant-negative effect, resulting in MMR deficiency even in the presence of the wild-type allele of the corresponding MMR gene and all other genes involved in the MMR process. To distinguish between these possibilities, the truncated polypeptide encoded by the *hP MS2-134* mutation was expressed in an MMR proficient cell line its affect on MMR activity was analyzed. The results showed that this mutant could indeed exert a dominant-negative effect, resulting in biochemical and genetic manifestations of MMR deficiency. One embodiment of the present invention is demonstrated in Table 1, where a Syrian hamster fibroblast cell line (TK) was transfected with an expression vector containing the hPMS2-134 (TK-PMS2-134) or the empty expression vector (TKvect), which also contains the NEO gene as a selectable marker. TK-PMS2-134 cells were determined to be stably expressing the gene via western blot analysis (data not shown). Nuclear lysates from hPMS2-134 and control cells were measured for the ability to correct mismatched DNA substrates. As shown in Table 1, TK-PMS2-134 cells had a dramatic decrease in repair activity while TKvect control cells were able to repair mismatched DNA duplexes at a rate of ∼4.0 fmol/15 minutes (p<.01).

**Table 1: Relative endogenous MMR activity of MMR-proficient cells expressing an ectopicaliy expressed morphogene or an empty expression vector**

| Cell Lines | 5' DNA Repair activity of G/T mismatch (fmol/15 minutes) |
|---|---|
| TKvect | |
| 1 | 3.5 |
| 2 | 2.9 |
| 3 | 5.5 |
| TK-PMS2-134 | |
| 1 | 0 |
| 2 | 0 |
| 3 | 0.5 |

These data show that the expression of the TK-PMS2-134 results in suppressed MMR of a host organism and allows for an enhanced mutation rate of genetic loci with each mitosis.

### Example 2: hPMS2-134 Can Alter Genes In Vivo

An example of the ability to alter mismatch repair comes from experiments using manipulation of mismatch repair TK cells (described above) that expressed the TK-hPMS2-134 mutant were used by transfection of the mammalian expression construct containing a defective β-galactosidase gene (referred to as pCAR-OF) which was transfected into TK-hPMS2-134 or TKvect cells as described above. The pCAR-OF vector consists of a β-galactosidase gene containing a 29-basepair poly-CA tract inserted at the 5' end of its coding region, which causes the wild-type reading frame to shift out-of-frame. This chimeric gene is cloned into the pCEP4, which contains the constitutively active cytomegalovirus (CMV) promoter upstream of the cloning site and also contains the hygromycin-resistance gene that allows for selection of cells containing this vector. The pCAR-OF reporter cannot generate β-galactosidase activity unless a frame-restoring mutation (i.e., insertion or deletion) arises following transfection into a host Another reporter vector called pCAR-IF contains a β-galactosidase in which a 27-bp poly-CA repeat was cloned into the same site as the pCAR-OF gene, but it is biologically active because the removal of a single repeat restores the open reading frame and produces a functional chimeric β-galactosidase polypeptide (not shown). In these experiments, TK-hPMS2-134 and TKvect cells were transfected with the pCAR-OF reporter vector and selected for 17 days in neomycin plus hygromycin selection medium. After the 17 days, resistant colonies were stained for β-galactosidase production to determine the number of clones containing a genetically altered β-galactosidase gene. All conditions produced a relatively equal number of neomycin/hygromycin resistant cells, however, only the cells expressing the TK-hPMS2-134 contained a subset of clones that were positive for β-galactosidase activity. Representative results are shown in Table 2, which shows the data from these experiments where cell colonies were stained *in situ* for β-galactosidase activity and scored-for activity. Cells were scored positive if the colonies turned blue in the presence of X-gal substrate and scored negative if colonies remained white. Analysis of triplicate experiments showed that a significant increase in the number of functional β-galactosidase positive cells was found in the TK-hPMS2-134 cultures, while no β-galactosidase positive cells were seen in the control TKvect cells.

**Table 2. Number of TKmorph and TKvect cells containing functional β-galactosidase activity**

| Cells B-gal | White Colonies | Blue Colonies | % Clones with altered |
|---|---|---|---|
| Tkvect | 65 +/- 9 | 0 | 0/65 = 0% |
| TK-PMS2-134 | 40+/-12 | 28 +/- 4 | 28/68 = 41% |

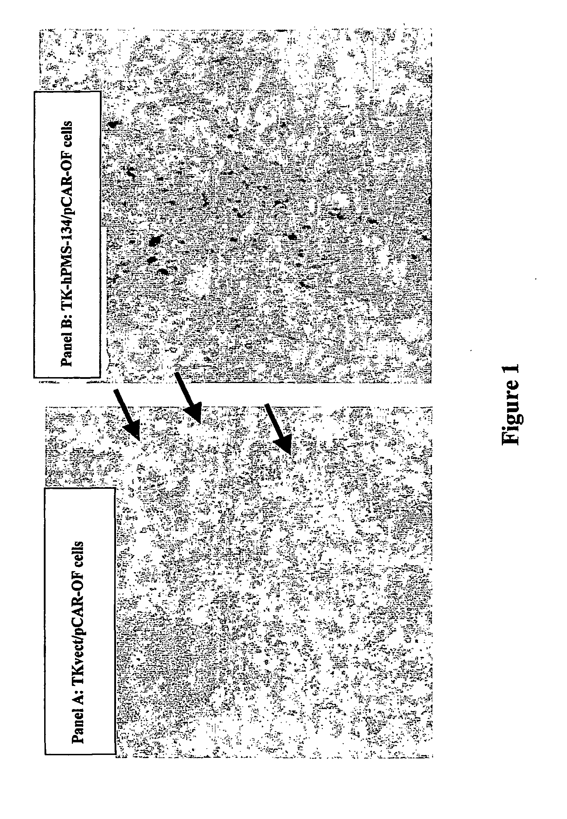

TK-PMS2-134/pCAR-OF clones that were pooled and expanded also showed a number of cells that contained a functional β-galactosidase gene. No β-galactosidase positive cells were observed in TKvect cells transfected with the pCAR-OF vector. These data are shown in Figure 1 where the dark staining in panel B represent β-galactosidase positive cells present in the TK-PMS2-134/pCAR-OF cultures while none are found in the TKvect cells grown under similar conditions (panel A). These data demonstrate the ability of the mutant mismatch repair gene, hPMS2-134, to generate gene alterations *in vivo,* which allows for the rapid screening of clones with altered polypeptides exhibiting new biochemical features.

To confirm that alterations within the nucleotide sequences of the β-galactosidase gene was indeed responsible for the *in vivo* β-galactosidase activity present in TK-hPMS2-134 clones, RNA was isolated from TK-hPMS2-134/pCAR OF and TKvect/pCAR-OF and the β-galactosidase mRNA primary structure was examined by reverse transcriptase polymerase chain reaction (RT-PCR) amplification and sequencing. Sequence analysis of β-galactosidase message from TKvect cells found no structural alterations in the input gene sequence. Analysis of the β-galactosidase message from TK-hPMS-134 cells found several changes within the coding sequences of the gene. These sequence alterations included insertion and deletions of the poly CA tract in the amino terminus as expected. Other alterations included insertions of sequences outside of the polyCA repeat as well as a series of single base alterations contained throughout the length of the gene.

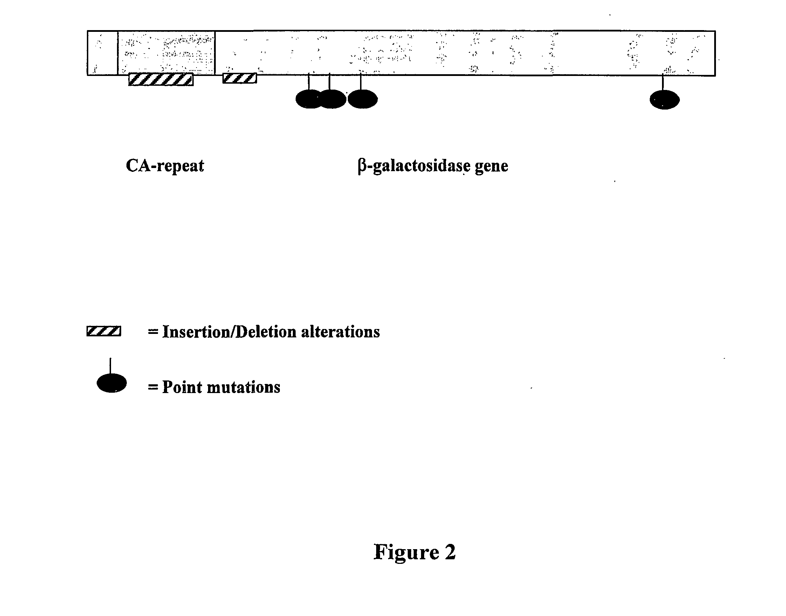

A summary of the genetic alterations are given in Figure 2 where a schematic representation of the β-galactosidase gene is shown with the regions and types of genetic alterations depicted below.

*Plasmids.* The full-length wild-type *hPMS2* cDNA was obtained from a human Hela cDNA library as described in Strand et. al., Nature, 1993, 365, 274-276.

An *hPMS2* cDNA containing a termination codon at amino acid 134 was obtained via RT-PCR from the patient in which the mutation was discovered. Nicolaides et. al., Mol. Cell. Biol., 1998, 18, 1635-1641.

The cDNA fragments were cloned into the BamHI site into the pSG5 vector, which contains an SV40 promoter followed by an SV40 polyadenylation signal. Nicolaides et al., Genomics, 1995, 29, 329-334. The pCAR reporter vectors described in Figure 1 were constructed as described in Palombo et al., Nature, 1994, 36, 417.

*β-galactosidase assay.* Seventeen days following transfection with pCAR, β-galactosidase assays were performed using 20 µg of protein in 45 mM 2-mercaptoethanol, 1 mM MgCl₂, 0.1 M NaPO₄ and 0.6 mg/ml Chlorophenol red-β-D-galatopyranoside (CPRG, Boehringer Mannheim). Reactions were incubated for 1 hour, terminated by the addition of 0.5 M Na₂CO₃, and analyzed by spectrophotometry at 576 nm. Nicolaides et. al., Mol. Cell. Biol., 1998, 18, 1635-1641. For *in situ* β-galactosidase staining, cells were fixed in 1% glutaraldehyde in PBS and incubated in 0.15 M NaCl, 1 mM MgCl₂, 3.3 mM K₄Fe(CN)₆, 3.3 mM K₃Fe(CN)₆, 0.2% X-Gal for 2 hours at 37°C. Example 3: hPMS2-134 Causes a Defect in MMR Activity

The differences in β-galactosidase activity between *PMS2-WT* and *PMS2-134* transfected cells can be due to the PMS2-134 protein disturbing MMR activity resulting in a higher frequency of mutation within the pCAR-OF reporter and re-establishing the ORF. To directly test whether MMR was altered, a biochemical assay for MMR with the individual clones described in Example 1 was employed. Nuclear extracts were prepared from the clones and incubated with heteroduplex substrates containing either a /CA\ insertion-deletion or a G/T mismatch under conditions described previously. The /CA\ and G/T heteroduplexes were used to test repair from the 3' and 5' directions, respectively. There was a dramatic difference between the *PMS2-134* expressing clones and the other clones in these assays (Table 3).

**Table 3. MMR activity of nuclear extracts from SH clones or pooled cultures^{a}**

| Cell line | | Amt of repaired substrate (fmol/15 min) | | | | |
|---|---|---|---|---|---|---|
| | | 3' /CA\ | 3' G/T | 5' G/T | 3' /CTG\ | 5' /CTG\ |
| SH clones*^{b}* | | | | | | |
| PMS2-NOT | | | | | | |
| Clone A | | 10.2 | | 3.5 | | |
| Clone B | | 12.7 | | 2.9 | | |
| Clone C | | 13.5 | | 5.5 | | |

| PMS2-WT | | | | | | |
|---|---|---|---|---|---|---|
| | Clone A | 2.8 | | 2.2 | | |
| | Clone B | 5.7 | | 4.8 | | |
| | Clone c | 4.7 | | 2.9 | | |

| PMS2-134 | | | | | | |
|---|---|---|---|---|---|---|
| | Clone A | 2.5 | | 0.0 | | |
| | Clone B | 2.4 | | 0.0 | | |
| | Clone C | 5.0 | | 0.5 | | |

| Pooled cultures | | | | | | |
|---|---|---|---|---|---|---|
| | PMS2-NOT | | 2.07 ± 0.09 | 2.37 ± 0.37 | 3.45 ± 1.35 | 2.77 ± 1.37 |
| | PMS2-WT | | 1.65 ± 0.94 | 1.86 ± 0.57 | 1.13 ± 0.23 | 1.23 ± 0.65 |
| | PMS2-134 | | 0.14 ± 0.2 | 0.0 ± 0.0 | 1.31 ± 0.66 | 0.0 ± 0.0 |

| | | | | | | |
|---|---|---|---|---|---|---|
| *^{a}* The extracts were tested for MMR activity with 24 fmol of heteroduplex. *^{b}* These data represent similar results derived from more than five independent experiments. | | | | | | |

While all clones repaired substrates from the 3' direction (/CA\ heteroduplex), cells expressing the PMS2-134 polypeptide had very little 5' repair activity. A similar directional defect in mismatch repair was evident with pooled clones resulting from *PMS2-134* transfection, or when the heteroduplex contained a 2-4 base pair loop, examples of which are shown in Table 3. A small decrease in MMR activity was observed in the 3' /CA\ PMS2-WT repair assays, perhaps a result of interference in the biochemical assays by over-expression of the PMS2 protein. No significant activity was caused by *PMS2-WT* in the *in situ* β-galactosidase assays, a result more likely to reflect the *in vivo* condition.

*Biochemical assays for mismatch repair.* MMR activity in nuclear extracts was performed as described, using 24 fmol of substrate, in Bronner et. al., Nature, 1994, 368, 258-261 and Nicolaides et. al., Mol. Cell. Biol., 1998, 18, 1635-1641.

Complementation assays were done by adding ∼ 100 ng of purified MutLα or MutSα components to 100 µg of nuclear extract, adjusting the final KCl concentration to 100 mM. Bevins, Ciba Found Symp., 1994, 186, 250-69 and Alderson et al., Res. Microbial., 1993, 144, 665-72. The substrates used in these experiments contain a strand break 181 nucleotides 5' or 125 nucleotides 3' to the mismatch. Values represent experiments performed at least in duplicate.

### Example 4: C-Terminus of hPMS2 Mediates Interaction Between hPMS2 and hMLH1

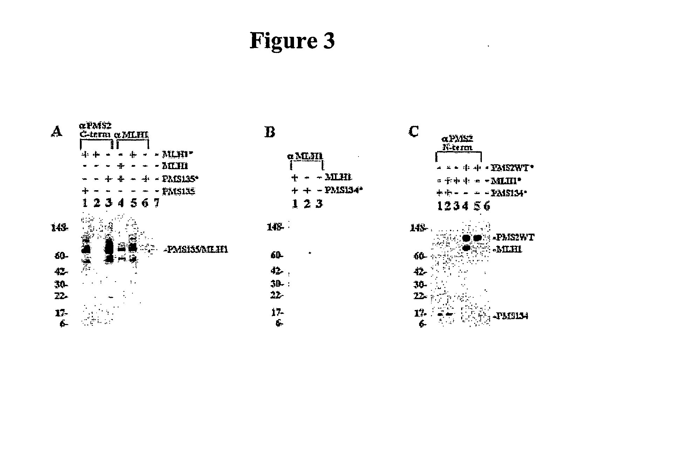

To elucidate the mechanism by which hPMS2-134 affected MMR, the interaction between hPMS2 and hMLH1 was analyzed. Previous studies have shown that these two proteins dimerize to form a functionally active complex. Bronner et. al., Nature, 1994, 368, 258-261. Proteins were synthesized *in vitro* using reticulocyte lysates, employing RNA generated from cloned templates. The full-length hMLH1 and hPMS2 proteins bound to each other and were co-precipitated with antibodies to either protein, as expected (data not shown). To determine the domain of hPMS2 that bound to hMLH1, the amino terminus (codons 1-134), containing the most highly conserved domain among mutL proteins (Su et al., J. Biol. Chem., 1988, 263, 6829-6835 and Edelmann et al., Cell, 1996, 85, 1125-1134), and the carboxyl terminus (codons 135-862) were separately cloned and proteins produced *in vitro* in coupled transcription-translation reactions. Figures 3A, 3B, and 3C show a representative immunoprecipitation of *in vitro*-translated hPMS2 and hMLH1 proteins. Figure 3A shows labeled (indicated by an asterisk) or unlabelled proteins incubated with an antibody to the C-terminus of hPMS2 in lanes 1 to 3 and to hMLH1 in lanes 4 to 6. Lane 7 contains a nonprogrammed reticulocyte lysate. PMS2-135 contains codons 135 to 862 of hPMS2. The major translation products of hPMS2 and hMLH1 are indicated. Figure 3B shows labeled hPMS2-134 (containing codons 1-134 of hPMS2) incubated in the presence or absence of unlabelled hMLH1 plus an antibody to hMLH1 (lanes 1 and 2, respectively). Lane 3 contains lysate from a nonprogrammed reticulolysate. Figure 3C shows labeled proteins incubated with an antibody to the N terminus of hPMS2. Lane 6 contains a nonprogrammed reticulocyte lysate. In both panels A and B, autoradiographs of immunoprecipitated products are shown. When a ³⁵S-labelled, full-length hMLH1 protein (Figure 3A, lane 5) was mixed with the unlabelled carboxyl terminal hPMS2 polypeptide, a monoclonal antibody (mAb) to the carboxyl terminus of hPMS2 efficiently immunoprecipitated the labeled hMLH1 protein (lane 1). No hMLH1 protein was precipitated in the absence of hPMS2 (lane 2). Conversely, when the ³⁵S-labelled carboxyl-terminus of hPMS2 (lane 3) was incubated with unlabelled, full-length hMLH1 protein, an and-hMLH1 mAb precipitated the hPMS2 polypeptide (lane 4). In the absence of the unlabelled hMLH1 protein, no hPMS2 protein was precipitated by this mAb (lane 6). The same antibody failed to immunoprecipitate the amino-terminus of hPMS2 (amino acids 1-134) when mixed with unlabelled MLH1 protein (Figure 3B, lane 1). This finding was corroborated by the converse experiment in which radiolabelled hPMS2-134 (Figure 3C, lane 1) was unable to coprecipitate radiolabelled MLH1 when precipitations were done using an N-terminal hPMS2 antibody (Figure 3C, lane 2) while this antibody was shown to be able to coprecipitate MLH1 when mixed with wild-type hPMS2 (Figure 3C, lane 4).

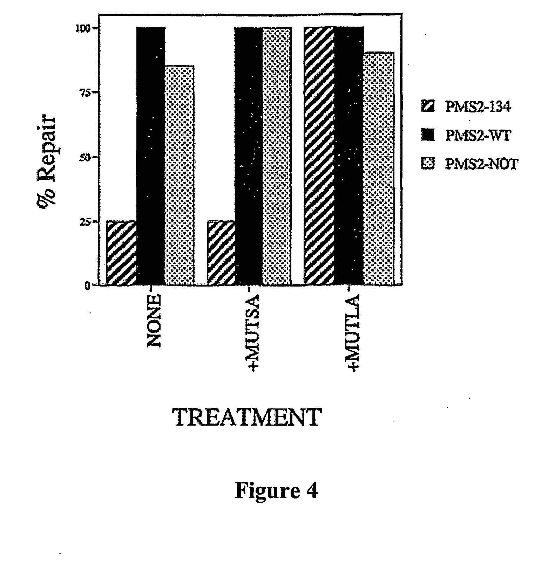

The initial steps of MMR are dependent on two protein complexes, called MutSα and MutLα. Drummond et. al., Science, 1995, 268, 1909-1912. As the amino terminus of hPMS2 did not mediate binding of hPMS2 to hMLH1, it was of interest to determine whether it might instead mediate the interaction between the MutLα complex (comprised of HULH1 and hPMS2) and the MutSα complex (comprised of MSH2 and GTBP). Because previous studies have demonstrated that MSH2 and the MutLα components do not associate in solution, direct hPMS2-134:MutSα interaction was unable to be assayed. A different approach was used to address this issue, and attempted to complement nuclear extracts from the various SH cell lines with MutSα or MutLα. If the truncated protein present in the PMS2-134 expressing SH cells was binding to MutSα and lowering its effective concentration in the extract, then adding intact MutSα should rescue the MMR defect in such extracts. Figure 4 shows complementation of MMR activity in transduced SH cells. Lysates from pooled clones stably transduced with PMS2-NOT, PMS2-WT, or PMS2-134 were complemented with purified MutSα or MutLα MMR components by using the 5' G/T heteroduplex substrate. The values are presented as the percentage of repair activity in each case compared to that in lysates complemented with both purified MutLα and MutSα components to normalize for repair efficiency in the different lysate backgrounds. The values shown represent the average of at least three different determinations. Purified MutSα added to such extracts had no effect (Figure 4). In contrast, addition of intact MutLα to the extract completely restored directional repair to the extracts from PMS2-134 cells (Figure 4).

The results described above lead to several conclusions. First, expression of the amino-terminus of hPMS2 results in an increase in microsatellite instability, consistent with a replication error (RER) phenotype. That this elevated microsatellite instability is due to MMR deficiency was proven by evaluation of extracts from stably transduced cells. Interestingly, the expression of PMS2-134 resulted in a polar defect in MMR, which was only observed using heteroduplexes designed to test repair from the 5' direction (no significant defect in repair from the 3' direction was observed in the same extracts). Interestingly, cells deficient in hMLH1 also have a polar defect in MMR, but in this case preferentially affecting repair from the 3' direction. Huttner et al., Pediatr. Res., 1999, 45, 785-94. It is known from previous studies in both prokaryotes and eukaryotes that the separate enzymatic components mediate repair from the two different directions. Our results indicate a model in which 5' repair is primarily dependent on hPMS2 while 3' repair is primarily dependent on hMLH1. It is easy to envision how the dimeric complex between PMS2 and MLH1 might set up this directionality. The combined results also demonstrate that a defect in directional MMR is sufficient to produce a RER+ phenotype.

The dominant-negative function of the PMS2-134 polypeptide can result from its binding to MLH1 and consequent inhibition of MutLα function. This is based in part on the fact that the most highly conserved domain of the *PMS2* gene is located in its amino terminus, and the only known biochemical partner for PMS2 is MLH1. The binding studies revealed, however, that the carboxyl terminus-of PMS2, rather than the highly conserved amino terminus, actually mediated binding to MLH1. This result is consistent with those recently obtained in *S. cerevisciae,* in which the MLH1-interacting domain of PMS1 (the yeast homolog of human PMS2) was localized to its carboxyl-terminus. Leach et al., Cell, 1993, 75, 1215-1225. The add-back experiments additionally showed that the hPMS2-134 mutant was not likely to mediate an interaction with the MutSα complex (Figure 4). The bPMS2-134 polypeptide does not inhibit the initial steps in MMR, but rather interacts with and inhibits a downstream component of the pathway, perhaps a nuclease required for repair from the 5' direction.

The demonstration that the hPMS2-134 mutation can confer a dominant-negative MMR defect to transfected cells helps to explain the phenotype of the kindred in which this mutant was discovered. Three individuals from this kindred were found to carry the mutation, a father and his two children. Both children exhibited microsatellite instability in their normal tissues and both developed tumors at an early age, while the father had no evidence of microsatellite instability in his normal cells and was completely healthy at age 35. The only difference known to us with respect to the MMR genes in this family is that the father's mutant allele was expressed at lower levels than the wild-type allele as assessed by sequencing of RT-PCR products of RNA from lymphocytes. The children expressed both alleles at approximately equal levels. The dominant negative attribute of the *hPMS2-134* mutant may only be manifest when it is present at sufficient concentrations (at least equimolar) thus, explaining the absence of MMR deficiency in the father. The reason for the differential expression of the *hPMS2-134* allele in this kindred is not clear, though imprinting is a possibility. Ascertainment of additional, larger kindreds with such mutations will facilitate the investigation of this issue.

*Western blots.* Equal number of cells were lysed directly in lysis buffer (60 mM Tris, pH 6.8, 2% SDS, 10% glycerol, 0.1 M 2-mercaptoethanol, 0.001% bromophenol blue) and boiled for 5 minutes. Lysate proteins were separated by electrophoresis on 4-12% Tris-glycine gels (for analysis of full-length hPMS2) or 4-20% Tris-glycine gels (for analysis of hPMS2-134). Gels were electroblotted onto Immobilon-P (Millipore) in 48 mM Tris base, 40 mM glycine, 0.0375% SDS, 20% methanol and blocked overnight at 4°C in Tris-buffered saline plus 0.05% Tween-20 and 5% condensed milk. Filters were probed with a polyclonal antibody generated against residues 2-20 of hPMS2 (Santa Cruz Biotechnology, Inc.) and a horseradish peroxidase conjugated goat anti-rabbit secondary antibody, using chemiluminescence for detection (Pierce).

*In vitro translation.* Linear DNA fragments containing *hPMS2* and *hMLH1* cDNA sequences were prepared by PCR, incorporating sequences for *in vitro* transcription and translation in the sense primer. A full-length *hMLH1* fragment was prepared using the sense primer 5'-ggatcctaatacgactcactatagggaga ccaccatgtcgttcgtggcaggg-3' (SEQ ID NO:1) (codons 1-6) and the antisense primer 5'-taagtcttaagtgctaccaaa-3' (SEQ ID NO:2) (located in the 3' untranslated region, nt 2411-2433), using a wild-type hMLH1 cDNA clone as template. A full-length *hPMS2* fragment was prepared with the sense primer 5'-ggatcctaatacgactcactatagggag accaccatggaacaattgcctgcgg-3' (SEQ ID NO:3) (codons 1- 6) and the antisense primer 5'-aggttagtgaagactctgtc-3' (SEQ ID NO:4) (located in 3' untranslated region, nt 2670-2690) using a cloned *hPMS2* cDNA as template. A fragment encoding the amino-terminal 134 amino acids of hPMS2 was prepared using the same sense primer and the antisense primer 5'-agtcgagttccaaccttcg-3' (SEQ ID NO:5). A fragment containing codons 135-862 of *hPMS135* was generated using the sense primer 5 '-ggatcetaatacgactcactatagggagaccaccatgatgtttgatcacaat gg-3' (SEQ ID NO:6) (codons 135-141) and the same antisense primer as that used for the full-length *hPMS2* protein. These fragments were used to produce proteins via the coupled transcription-translation system (Promega). The reactions were supplemented with ³⁵S-labelled methionine or unlabelled methionine, as indicated in the text. The PMS135 and hMLH1 proteins could not be simultaneously radiolabelled and immunoprecipitated because of their similar molecular weights precluded resolution. Lower molecular weight bands are presumed to be degradation products and/or polypeptides translated from alternative internal methionines.

*Immunoprecipitation*. Immunoprecipitations were performed on *in vitro* translated proteins by mixing the translation reactions with 1 µg of the MLH1 specific monoclonal antibody (mAB) MLH14 (Oncogene Science, Inc.), a polyclonal antibody generated to codons 2-20 of hPMS2 described above, or a polyclonal antibody generated to codons 843-862 of hPMS2 (Santa Cruz Biotechnology, Inc.) in 400 µl of EBC buffer (50 mM Tris, pH 7.5, 0.1 M NaCl, 0.5% NP40). After incubation for 1 hour at 4°C, protein A sepharose (Sigma) was added to a final concentration of 10% and reactions were incubated at 4°C for 1 hour. Proteins bound to protein A were washed five times in EBC and separated by electrophoresis on 4-20% Tris-glycine gels, which were then dried and autoradiographed.

### Example 5: Syrian hamster Tk-ts-13 Cells Produce a Novel Anti-Microbial Polypeptide Can Suppress the Growth of Bacillus subtilis

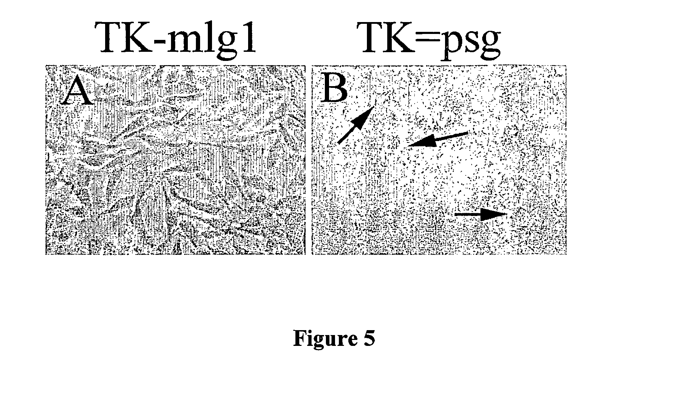

The feasibility of creating microbial-resistant mammalian cells is demonstrated as follows. Syrian Hamster TK fibroblasts were transfected with a mammalian expression vector containing a novel anti-microbial polypeptide called mlg1 or the empty expression vector called psg. When cells expressing the mlg polypeptide (referred to as TK-mlg1) were grown in the presence of *Bacillus subtilis,* these cells were able to suppress the growth of the microbes and allow the TK host to remain viable in contrast to TK cells transfected with the empty vector (TK=psg), which all died from the toxic effects that *Bacillus subtilis* has on mammalian cells. Figure 5 shows a photograph of TK-mlg1 and TK=psg cultures grown in the presence of *Bacillus* for 4 days. Syrian hamster Tk-ts 13 cells were transfected with a eukaryotic expression vector that produces a novel antimicrobial polypeptide referred to as mlg1 (Panel A) or the expression vector lacking an inserted cDNA for expression (TK=psg, Panel B). Cells were plated at a density of 5x10⁵ cells/ml in a 10 cm falcon pyrogenic-free petri dish in growth medium for 24 hours and then inoculated with 10 µl of an exponentially growing culture of *Bacillus subtilis.* Cultures were then incubated for 4 days at which time *Bacilli* grow and begin to lyse the Tk-ts13 parental culture as shown in panel B (indicated by arrows), while cells expressing the anti-microbial mlg1 polypeptide (Panel A) remain viable in the presence of *Bacillus* (small granular structures present in panels A and B). These data demonstrate the feasibility of cells to survive in the presence of *Bacillus* contamination when they produce an anti-microbial agent These data show that antimicrobial producing mammalian cells are capable of growing and surviving in the presence of toxic microbes.

*Cell lines and trarrsfection.* Syrian Hamster fibroblast Tk-ts13 cells were obtained from ATCC and cultured as described. Modrich, Science, 1994, 266, 1959-1960. Stably transfected cell lines expressing *hPMS2* were created by cotransfection of the *PMS2* expression vectors and the pLHL4 plasmid encoding the hygromycin resistance gene at a ratio of 3:1 (pCAR:pLHL4) and selected with hygromycin. Stably transfected cell lines containing pCAR reporters were generated by co-transfection of pCAR vectors together with either pNTK plasmid encoding the neomycin resistance plasmid or with pLHL4. All transfections were performed using calcium phosphate as previously described in Modrich, Science, 1994, 266, 1959-1960.

### Example 6: TK-hPMS2-134 Cells Can Suppress the Growth of Escherichia coli in vitro

While TK-hPMS2-134 TK-ts13 cells have been previously shown to be capable of altering genes *in vivo* (refer to Table 2 and Figure 1), the ability to generate "naturally microbial-resistant" clones has not been reported in the literature. To generate microbial-resistant TK cells, TK-ts13 cells constitutively expressing the a dominant-negative mismatch repair gene, TK-hPMS2-134 or the empty vector (TKvect) that have been in culture for >3 months (∼60 passages) were seeded at 5x10⁵ cells/ml in Dulbelcco's Modified Eagles Medium (DMEM) plus 10% fetal bovine serum (FBS) and plated into 10 cm dishes (Falcon) in duplicate. These cells were grown overnight at 37°C in 5%CO₂ to allow cells to adhere to the plastic. The next day, TK cultures were inoculated with 10 µl of an exponentially growing culture of *Escherichia coli.* Cultures were then grown at 37°C in 5%CO₂ and observed on day 7 and 14 for microbial-resistant cell clones; these cells appear as clones of cells surrounded by "cleared" areas on the plate. At day 7, all cells in the control transfected TKvect culture were dead, while a subset of cells were viable in the TK-hPMS2-134 transfected cultures. At day 14, there were no clones in the control transfected TKvect cultures, while there were 34 and 40 *Escherichia coli*-resistant colonies formed in the TK-hPMS2-134 transfected cultures. Growing clones from each dish were then pooled as individual cultures and grown to confluence. These cultures were named TK-hPMS2-134 (R1) and TK-hPMS2-134 (R2). Cultures were cured of *Escherichia coli* by the addition of 1 mg/ml G418, in which the TK-hPMS2-134 cells are resistant due to expression of the neomycin-resistance gene contained on the mammalian expression vector used to generate the cells.

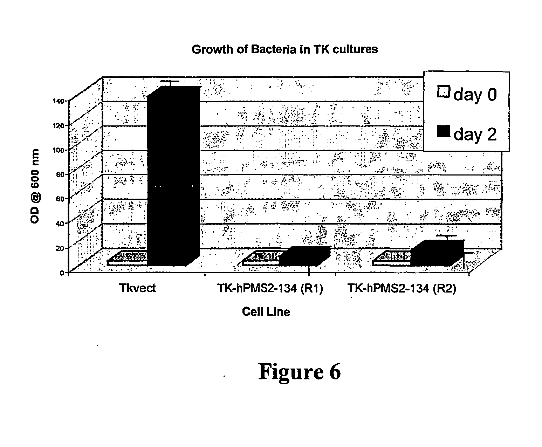

TKvect, TK-hPMS2-134 (R1) and TK-hPMS2-134 (R2) cells were plated at 5x10⁵ cell/ml in 10 mls and plated into 10 cm dishes in duplicate. The next day, 10 µl of a logarithmic stage *Escherichia coli* culture was added to each TK culture and cultures were grown for 48 hours at 37°C in 5%CO₂. An aliquot of supernatant from each culture was harvested immediately after inoculation to establish a baseline density of bacteria for each culture. After 48 hours, 2 ml of supernatant were harvested from each culture as well as from uninfected TK cultures. One ml of each supernatant was then analyzed by a spectrophotometer at an OD₆₀₀ to measure for bacterial density. Supernatants from uninfected cultures were used as a blank to correct for background. As shown in Figure 6, bacterial growth was significantly suppressed in TK-hPMS2-134 (R1) and TK-hPMS2-134 (R2) cultures in contrast to TKvect control cells. These data demonstrate the feasibility of using a dominant-negative mismatch repair mutant hPMS2-134 on mammalian cells to produce genetically altered clones capable of producing a molecule(s) that can suppress microbial growth.

As those skilled in the art will appreciate, numerous changes and modifications may be made to the preferred embodiments of the invention,

## Claims

1. A method for obtaining a mammalian cell that is resistant to a selected microbe, comprising:
growing a culture of mammalian cells wherein said cells comprise hPMS2-134;
exposing said cells to said selected microbe; and
selecting said mammalian cell that is resistant to said selected microbe.

2. A method according to claim 1, wherein said step of selecting for microbial resistance comprises isolating and testing culture medium from said cell.

3. A method for obtaining a cell that comprises a mutation in a gene encoding microbial resistance, comprising:
growing a culture of mammalian cells comprising said gene encoding said microbial resistance and hPMS2-134;
selecting a cell having said microbial resistance; and
determining whether said gene comprises a mutation.

4. A method according to claim 1 or 3, wherein said cell is selected for resistance to a bacterium.

5. A method according to claim 1 or 3, wherein said cell is selected for resistance to a fungus.

6. A method according to claim 3, wherein said step of selecting a cell having said microbial resistance comprises isolating and testing culture medium from said cell.

7. A method according to claim 3, wherein said step of determining whether said gene comprises a mutation comprises analyzing a nucleotide sequence of said gene.

8. A method according to claim 3, wherein said step of determining whether said gene comprises a mutation comprises analyzing mRNA transcribed from said gene.

9. A method according to claim 3, wherein said step of determining whether said gene comprises a mutation comprises analyzing a protein encoded by said gene.

10. A method according to claim 3, wherein said step of determining whether said gene comprises a mutation comprises analyzing a phenotypic trait determined by said gene.

## Patentansprüche

1. Verfahren zur Gewinnung einer Säugerzelle, die gegenüber einer ausgewählten Mikrobe resistent ist, das Folgendes umfasst:
Wachsen einer Kultur von Säugerzellen, worin die genannten Zellen hPMS2-134 umfassen;
Exponieren der genannten Zellen gegenüber der genannten ausgewählten Mikrobe; und
Auswählen der genannten Säugerzelle, die gegenüber der genannten ausgewählten Mikrobe resistent ist.

2. Verfahren nach Anspruch 1, worin der genannte Schritt des Auswählens nach mikrobieller Resistenz das Isolieren und Testen des Kulturmediums aus der genannten Zelle umfasst.

3. Verfahren zur Gewinnung einer Zelle, die eine Mutation in einem Gen umfasst, das mikrobielle Resistenz kodiert, das Folgendes umfasst:
Wachsen einer Kultur von Säugerzellen, die das genannte Gen, das die genannte mikrobielle Resistenz kodiert, und hPMS2-134 umfassen;
Auswählen einer Zelle, die die genannte mikrobielle Resistenz aufweist; und
Bestimmen, ob das genannte Gen eine Mutation umfasst.

4. Verfahren nach Anspruch 1 oder 3, worin die genannte Zelle nach Resistenz gegenüber einem Bakterium ausgewählt wird.

5. Verfahren nach Anspruch 1 oder 3, worin die genannte Zelle nach Resistenz gegenüber einem Pilz ausgewählt wird.

6. Verfahren nach Anspruch 3, worin der genannte Schritt des Auswählens einer Zelle, die die genannte mikrobielle Resistenz aufweist, das Isolieren und Testen des Kulturmediums aus der genannten Zelle umfasst.

7. Verfahren nach Anspruch 3, worin der genannte Schritt des Bestimmens, ob das genannte Gen eine Mutation umfasst, das Analysieren einer Nukleotidsequenz des genannten Gens umfasst.

8. Verfahren nach Anspruch 3, worin der genannte Schritt des Bestimmens, ob das genannte Gen eine Mutation umfasst, das Analysieren von mRNA, die von dem genannten Gen transkribiert wird, umfasst.

9. Verfahren nach Anspruch 3, worin der genannte Schritt des Bestimmens, ob das genannte Gen eine Mutation umfasst, das Analysieren eines Proteins, das von dem genannten Gen kodiert wird, umfasst.

10. Verfahren nach Anspruch 3, worin der genannte Schritt des Bestimmens, ob das genannte Gen eine Mutation umfasst, das Analysieren eines phänotypischen Merkmals, das von dem genannten Gen bestimmt wird, umfasst.

## Revendications

1. Méthode d'obtention d'une cellule mammalienne qui est résistante à un microbe sélectionné, comprenant :
le développement d'une culture de cellules mammaliennes, où lesdites cellules comprennent hPMS2-134 ;
l'exposition desdites cellules audit microbe sélectionné ; et
la sélection de ladite cellule mammalienne qui est résistante audit microbe sélectionné.

2. Méthode selon la revendication 1, dans laquelle ladite étape de sélection pour une résistance microbienne comprend l'isolement et l'analyse du milieu de culture à partir de ladite cellule.

3. Méthode d'obtention d'une cellule qui comprend une mutation dans un gène codant pour une résistance microbienne, comprenant :
le développement d'une culture de cellules mammaliennes comprenant ledit gène codant pour ladite résistance microbienne et hPMS2-134 ;
la sélection d'une cellule ayant ladite résistance microbienne ; et
la détermination du fait que ledit gène comprend ou non une mutation.

4. Méthode selon la revendication 1 ou 3, dans laquelle ladite cellule est sélectionnée pour une résistance vis-à-vis d'une bactérie.

5. Méthode selon la revendication 1 ou 3, dans laquelle ladite cellule est sélectionnée pour une résistance vis-à-vis d'un champignon.

6. Méthode selon la revendication 3, dans laquelle ladite étape de sélection d'une cellule ayant ladite résistance microbienne comprend l'isolement et l'analyse du milieu de culture à partir de ladite cellule.

7. Méthode selon la revendication 3, dans laquelle ladite étape de détermination du fait que ledit gène comprend ou non une mutation, comprend l'analyse d'une séquence nucléotidique dudit gène.

8. Méthode selon la revendication 3, dans laquelle ladite étape de détermination du fait que ledit gène comprend ou non une mutation, comprend l'analyse d'ARNm transcrit à partir dudit gène.

9. Méthode selon la revendication 3, dans laquelle ladite étape de détermination du fait que ledit gène comprend ou non une mutation, comprend l'analyse d'une protéine codée par ledit gène.

10. Méthode selon la revendication 3, dans laquelle ladite étape de détermination du fait que ledit gène comprend ou non une mutation, comprend l'analyse d'un caractère phénotypique déterminé par ledit gène.


### SEQUENCE LISTING

<110> Nicolaides, Nicholas C
   Grasso, Luigi
   Sass, Philip M
<120> METHODS FOR ISOLATING NOVEL ANTIMICROBIAL AGENTS FROM
   HYPERMUTABLE CELLS
<130> MOR0006
<140> 00/000,000
   <141> 2000-11-07
<160> 16
<170> PatentIn Ver. 2.1
<210> 1
   <211> 52
   <212> DNA
   <213> Artificial Sequence
<220>
   <223> Description of Artificial Sequence:oligonucleotide primer
<400> 1
   ggatcctaat acgactcact atagggagac caccatgtcg ttcgtggcag gg 52
<210> 2
   <211> 21
   <212> DNA
   <213> Artificial Sequence
<220>
   <223> Description of Artificial Sequence:oligonucleotide primer
<400> 2
   taagtcttaa gtgctaccaa c 21
<210> 3
   <211> 53
   <212> DNA
   <213> Artificial Sequence
<220>
   <223> Description of Artificial Sequence:oligonucleotide primer
<400> 3
   ggatcctaat acgactcact atagggagac caccatggaa caattgcctg cgg 53
<210> 4
   <211> 20
   <212> DNA
   <213> Artificial Sequence
<220>
   <223> Description of Artificial Sequence:oligonucleotide primer
<400> 4
   aggttagtga agactctgtc 20
<210> 5
   <211> 19
   <212> DNA
   <213> Artificial Sequence
<220>
   <223> Description of Artificial Sequence:primer
<400> 5
   agtcgagttc caaccttcg 19
<210> 6
   <211> 54
   <212> DNA
   <213> Artificial Sequence
<220>
   <223> Description of Artificial Sequence:primer
<400> 6
   ggatcctaat acgactcact atagggagac caccatgatg tttgatcaca atgg 54
<210> 7
   <211> 859
   <212> PRT
   <213> Mus musculus
<400> 7
   Met Glu Gln Thr Glu Gly Val Ser Thr Glu Cys Ala Lys Ala Ile Lys
    1               5                  10                  15
   Pro Ile Asp Gly Lys Ser Val His Gln Ile Cys Ser Gly Gln Val Ile
               20                  25                  30
   Leu Ser Leu Ser Thr Ala Val Lys Glu Leu Ile Glu Asn Ser Val Asp
           35                  40                  45
   Ala Gly Ala Thr Thr Ile Asp Leu Arg Leu Lys Asp Tyr Gly Val Asp
       50                  55                  60
   Leu Ile Glu Val Ser Asp Asn Gly Cys Gly Val Glu Glu Glu Asn Phe
   65                  70                  75                  80
   Glu Gly Leu Ala Leu Lys His His Thr Ser Lys Ile Gln Glu Phe Ala
                   85                  90                  95
   Asp Leu Thr Gln Val Glu Thr Phe Gly Phe Arg Gly Glu Ala Leu Ser
               100                 105                 110
   Ser Leu Cys Ala Leu Ser Asp Val Thr Ile Ser Thr Cys His Gly Ser
           115                 120                 125
   Ala Ser Val Gly Thr Arg Leu Val Phe Asp His Asn Gly Lys Ile Thr
       130                 135                 140
   Gln Lys Thr Pro Tyr Pro Arg Pro Lys Gly Thr Thr Val Ser Val Gln
   145                 150                 155                 160
   His Leu Phe Tyr Thr Leu Pro Val Arg Tyr Lys Glu Phe Gln Arg Asn
                   165                 170                 175
   Ile Lys Lys Glu Tyr Ser Lys Met Val Gln Val Leu Gln Ala Tyr Cys
               180                 185                 190
   Ile Ile Ser Ala Gly Val Arg Val Ser Cys Thr Asn Gln Leu Gly Gln
           195                 200                 205
   Gly Lys Arg His Ala Val Val Cys Thr Ser Gly Thr Ser Gly Met Lys
       210                 215                 220
   Glu Asn Ile Gly Ser Val Phe Gly Gln Lys Gln Leu Gln Ser Leu Ile
   225                 230                 235                 240
   Pro Phe Val Gln Leu Pro Pro Ser Asp Ala Val Cys Glu Glu Tyr Gly
   245 250 255
   Leu Ser Thr Ser Gly Arg His Lys Thr Phe Ser Thr Phe Arg Ala Ser
   260 265 270
   Phe His Ser Ala Arg Thr Ala Pro Gly Gly Val Gln Gln Thr Gly Ser
   275 280 285
   Phe Ser Ser Ser Ile Arg Gly Pro Val Thr Gln Gln Arg Ser Leu Ser
   290 295 300
   Leu Ser Met Arg Phe Tyr His Met Tyr Asn Arg His Gln Tyr Pro Phe
   305 310 315 320
   Val Val Leu Asn Val Ser Val Asp Ser Glu Cys Val Asp Ile Asn Val
   325 330 335
   Thr Pro Asp Lys Arg Gln Ile Leu Leu Gln Glu Glu Lys Leu Leu Leu
   340 345 350
   Ala Val Leu Lys Thr Ser Leu Ile Gly Met Phe Asp Ser Asp Ala Asn
   355 360 365
   Lys Leu Asn Val Asn Gln Gln Pro Leu Leu Asp Val Glu Gly Asn Leu
   370 375 380
   Val Lys Leu His Thr Ala Glu Leu Glu Lys Pro Val Pro Gly Lys Gln
   385 390 395 400
   Asp Asn Ser Pro Ser Leu Lys Ser Thr Ala Asp Glu Lys Arg Val Ala
   405 410 415
   Ser Ile Ser Arg Leu Arg Glu Ala Phe Ser Leu His Pro Thr Lys Glu
   420 425 430
   Ile Lys Ser Arg Gly Pro Glu Thr Ala Glu Leu Thr Arg Ser Phe Pro
   435 440 445
   Ser Glu Lys Arg Gly Val Leu Ser Ser Tyr Pro Ser Asp Val Ile Ser
   450 455 460
   Tyr Arg Gly Leu Arg Gly Ser Gln Asp Lys Leu Val Ser Pro Thr Asp
   465 470 475 480
   Ser Pro Gly Asp Cys Met Asp Arg Glu Lys Ile Glu Lys Asp Ser Gly
   485 490 495
   Leu Ser Ser Thr Ser Ala Gly Ser Glu Glu Glu Phe Ser Thr Pro Glu
               500                 505                 510
   Val Ala Ser Ser Phe Ser Ser Asp Tyr Asn Val Ser Ser Leu Glu Asp
           515                 520                 525
   Arg Pro Ser Gln Glu Thr Ile Asn Cys Gly Asp Leu Asp Cys Arg Pro
       530                 535                 540
   Pro Gly Thr Gly Gln Ser Leu Lys Pro Glu Asp His Gly Tyr Gln Cys
   545                 550                 555                 560
   Lys Ala Leu Pro Leu Ala Arg Leu Ser Pro Thr Asn Ala Lys Arg Phe
                   565                 570                 575
   Lys Thr Glu Glu Arg Pro Ser Asn Val Asn Ile Ser Gln Arg Leu Pro
               580                 585                 590
   Gly Pro Gln Ser Thr Ser Ala Ala Glu Val Asp Val Ala Ile Lys Met
           595                 600                 605
   Asn Lys Arg Ile Val Leu Leu Glu Phe Ser Leu Ser Ser Leu Ala Lys
       610                 615                 620
   Arg Met Lys Gln Leu Gln His Leu Lys Ala Gln Asn Lys His Glu Leu
   625                 630                 635                 640
   Ser Tyr Arg Lys Phe Arg Ala Lys Ile Cys Pro Gly Glu Asn Gln Ala
                   645                 650                 655
   Ala Glu Asp Glu Leu Arg Lys Glu Ile Ser Lys Ser Met Phe Ala Glu
               660                 665                 670
   Met Glu Ile Leu Gly Gln Phe Asn Leu Gly Phe Ile Val Thr Lys Leu
           675                 680                 685
   Lys Glu Asp Leu Phe Leu Val Asp Gln His Ala Ala Asp Glu Lys Tyr
       690                 695                 700
   Asn Phe Glu Met Leu Gln Gln His Thr Val Leu Gln Ala Gln Arg Leu
   705                 710                 715                 720
   Ile Thr Pro Gln Thr Leu Asn Leu Thr Ala Val Asn Glu Ala Val Leu
                   725                 730                 735
   Ile Glu Asn Leu Glu Ile Phe Arg Lys Asn Gly Phe Asp Phe Val Ile
               740                 745                 750
   Asp Glu Asp Ala Pro Val Thr Glu Arg Ala Lys Leu Ile Ser Leu Pro
           755                 760                     765
   Thr Ser Lys Asn Trp Thr Phe Gly Pro Gln Asp Ile Asp Glu Leu Ile
       770                 775                     780
   Phe Met Leu Ser Asp Ser Pro Gly Val Met Cys Arg Pro Ser Arg Val
   785                 790                 795                     800
   Arg Gln Met Phe Ala Ser Arg Ala Cys Arg Lys Ser Val Met Ile Gly
                   805                 810                     815
   Thr Ala Leu Asn Ala Ser Glu Met Lys Lys Leu Ile Thr His Met Gly
               820                 825                     830
   Glu Met Asp His Pro Trp Asn Cys Pro His Gly Arg Pro Thr Met Arg
           835                 840                     845
   His Val Ala Asn Leu Asp Val Ile Ser Gln Asn
       850                 855
<210> 8
   <211> 3056
   <212> DNA
   <213> Mus musculus
<400> 8
   gaattccggt gaaggtcctg aagaatttcc agattcctga gtatcattgg aggagacaga 60
   taacctgtcg tcaggtaacg atggtgtata tgcaacagaa atgggtgttc ctggagacgc 120
   gtcttttccc gagagcggca ccgcaactct cccgcggtga ctgtgactgg aggagtcctg 180
   catccatgga gcaaaccgaa ggcgtgagta cagaatgtgc taaggccatc aagcctattg 240
   atgggaagtc agtccatcaa atttgttctg ggcaggtgat actcagttta agcaccgctg 300
   tgaaggagtt gatagaaaat agtgtagatg ctggtgctac tactattgat ctaaggctta 360
   aagactatgg ggtggacctc attgaagttt cagacaatgg atgtggggta gaagaagaaa 420
   actttgaagg tctagctctg aaacatcaca catctaagat tcaagagttt gccgacctca 480
   cgcaggttga aactttcggc tttcgggggg aagctctgag ctctctgtgt gcactaagtg 540
   atgtcactat atctacctgc cacgggtctg caagcgttgg gactcgactg gtgtttgacc 600
   ataatgggaa aatcacccag aaaactccct acccccgacc taaaggaacc acagtcagtg 660
   tgcagcactt attttataca ctacccgtgc gttacaaaga gtttcagagg aacattaaaa 720
   aggagtattc caaaatggtg caggtcttac aggcgtactg tatcatctca gcaggcgtcc 780
   gtgtaagctg cactaatcag ctcggacagg ggaagcggca cgctgtggtg tgcacaagcg 840
   gcacgtctgg catgaaggaa aatatcgggt ctgtgtttgg ccagaagcag ttgcaaagcc 900
   tcattccttt tgttcagctg ccccctagtg acgctgtgtg tgaagagtac ggcctgagca 960
   cttcaggacg ccacaaaacc ttttctacgt ttcgggcttc atttcacagt gcacgcacgg 1020
   cgccgggagg agtgcaacag acaggcagtt tttcttcatc aatcagaggc cctgtgaccc 1080
   agcaaaggtc tctaagcttg tcaatgaggt tttatcacat gtataaccgg catcagtacc 1140
   catttgtcgt ccttaacgtt tccgttgact cagaatgtgt ggatattaat gtaactccag 1200
   ataaaaggca aattctacta caagaagaga agctattgct ggccgtttta aagacctcct 1260
   tgataggaat gtttgacagt gatgcaaaca agcttaatgt caaccagcag ccactgctag 1320
   atgttgaagg taacttagta aagctgcata ctgcagaact agaaaagcct gtgccaggaa 1380
   agcaagataa ctctccttca ctgaagagca cagcagacga gaaaagggta gcatccatct 1440
   ccaggctgag agaggccttt tctcttcatc ctactaaaga gatcaagtct aggggtccag 1500
   agactgctga actgacacgg agttttccaa gtgagaaaag gggcgtgtta tcctcttatc 1560
   cttcagacgt catctcttac agaggcctcc gtggctcgca ggacaaattg gtgagtccca 1620
   cggacagccc tggtgactgt atggacagag agaaaataga aaaagactca gggctcagca 1680
   gcacctcagc tggctctgag gaagagttca gcaccccaga agtggccagt agctttagca 1740
   gtgactataa cgtgagctcc ctagaagaca gaccttctca ggaaaccata aactgtggtg 1800
   acctggactg ccgtcctcca ggtacaggac agtccttgaa gccagaagac catggatatc 1860
   aatgcaaagc tctacctcta gctcgtctgt cacccacaaa tgccaagcgc ttcaagacag 1920
   aggaaagacc ctcaaatgtc aacatttctc aaagattgcc tggtcctcag agcacctcag 1980
   cagctgaggt cgatgtagcc ataaaaatga ataagagaat cgtgctcctc gagttctctc 2040
   tgagttctct agctaagcga atgaagcagt tacagcacct aaaggcgcag aacaaacatg 2100
   aactgagtta cagaaaattt agggccaaga tttgccctgg agaaaaccaa gcagcagaag 2160
   atgaactcag aaaagagatt agtaaatcga tgtttgcaga gatggagatc ttgggtcagt 2220
   ttaacctggg atttatagta accaaactga aagaggacct cttcctggtg gaccagcatg 2280
   ctgcggatga gaagtacaac tttgagatgc tgcagcagca cacggtgctc caggcgcaga 2340
   ggctcatcac accccagact ctgaacttaa ctgctgtcaa tgaagctgta ctgatagaaa 2400
   atctggaaat attcagaaag aatggctttg actttgtcat tgatgaggat gctccagtca 2460
   ctgaaagggc taaattgatt tccttaccaa ctagtaaaaa ctggaccttt ggaccccaag 2520
   atatagatga actgatcttt atgttaagtg acagccctgg ggtcatgtgc cggccctcac 2580
   gagtcagaca gatgtttgct tccagagcct gtcggaagtc agtgatgatt ggaacggcgc 2640
   tcaatgcgag cgagatgaag aagctcatca cccacatggg tgagatggac cacccctgga 2700
   actgccccca cggcaggcca accatgaggc acgttgccaa tctggatgtc atctctcaga 2760
   actgacacac cccttgtagc atagagttta ttacagattg ttcggtttgc aaagagaagg 2820
   ttttaagtaa tctgattatc gttgtacaaa aattagcatg ctgctttaat gtactggatc 2880
   catttaaaag cagtgttaag gcaggcatga tggagtgttc ctctagctca gctacttggg 2940
   tgatccggtg ggagctcatg tgagcccagg actttgagac cactccgagc cacattcatg 3000
   agactcaatt caaggacaaa aaaaaaaaga tatttttgaa gccttttaaa aaaaaa     3056
<210> 9
   <211> 862
   <212> PRT
   <213> Homo sapiens
<400> 9
   Met Glu Arg Ala Glu Ser Ser Ser Thr Glu Pro Ala Lys Ala Ile Lys
    1               5                  10                  15
   Pro Ile Asp Arg Lys Ser Val His Gln Ile Cys Ser Gly Gln Val Val
               20                  25                  30
   Leu Ser Leu Ser Thr Ala Val Lys Glu Leu Val Glu Asn Ser Leu Asp
           35                  40                  45
   Ala Gly Ala Thr Asn Ile Asp Leu Lys Leu Lys Asp Tyr Gly Val Asp
       50                  55                  60
   Leu Ile Glu Val Ser Asp Asn Gly Cys Gly Val Glu Glu Glu Asn Phe
   65                  70                  75                  80
   Glu Gly Leu Thr Leu Lys His His Thr Ser Lys Ile Gln Glu Phe Ala
                   85                  90                  95
   Asp Leu Thr Gln Val Glu Thr Phe Gly Phe Arg Gly Glu Ala Leu Ser
               100                 105                 110
   Ser Leu Cys Ala Leu Ser Asp Val Thr Ile Ser Thr Cys His Ala Ser
           115                 120                 125
   Ala Lys Val Gly Thr Arg Leu Met Phe Asp His Asn Gly Lys Ile Ile
       130                 135                 140
   Gln Lys Thr Pro Tyr Pro Arg Pro Arg Gly Thr Thr Val Ser Val Gln
   145                 150                 155                 160
   Gln Leu Phe Ser Thr Leu Pro Val Arg His Lys Glu Phe Gln Arg Asn
                   165                 170                 175
   Ile Lys Lys Glu Tyr Ala Lys Met Val Gln Val Leu His Ala Tyr Cys
               180                 185                 190
   Ile Ile Ser Ala Gly Ile Arg Val Ser Cys Thr Asn Gln Leu Gly Gln
           195                 200                 205
   Gly Lys Arg Gln Pro Val Val Cys Thr Gly Gly Ser Pro Ser Ile Lys
       210                 215                 220
   Glu Asn Ile Gly Ser Val Phe Gly Gln Lys Gln Leu Gln Ser Leu Ile
   225                 230                 235                 240
   Pro Phe Val Gln Leu Pro Pro Ser Asp Ser Val Cys Glu Glu Tyr Gly
                   245                 250                 255
   Leu Ser Cys Ser Asp Ala Leu His Asn Leu Phe Tyr Ile Ser Gly Phe
               260                 265                 270
   Ile Ser Gln Cys Thr His Gly Val Gly Arg Ser Ser Thr Asp Arg Gln
           275                 280                 285
   Phe Phe Phe Ile Asn Arg Arg Pro Cys Asp Pro Ala Lys Val Cys Arg
       290                 295                 300
   Leu Val Asn Glu Val Tyr His Met Tyr Asn Arg His Gln Tyr Pro Phe
   305 310 315 320
   Val Val Leu Asn Ile Ser Val Asp Ser Glu Cys Val Asp Ile Asn Val
   325 330 335
   Thr Pro Asp Lys Arg Gln Ile Leu Leu Gln Glu Glu Lys Leu Leu Leu
   340 345 350
   Ala Val Leu Lys Thr Ser Leu Ile Gly Met Phe Asp Ser Asp Val Asn
   355 360 365
   Lys Leu Asn Val Ser Gln Gln Pro Leu Leu Asp Val Glu Gly Asn Leu
   370 375 380
   Ile Lys Met His Ala Ala Asp Leu Glu Lys Pro Met Val Glu Lys Gln
   385 390 395 400
   Asp Gln Ser Pro Ser Leu Arg Thr Gly Glu Glu Lys Lys Asp Val Ser
   405 410 415
   Ile Ser Arg Leu Arg Glu Ala Phe Ser Leu Arg His Thr Thr Glu Asn
   420 425 430
   Lys Pro His Ser Pro Lys Thr Pro Glu Pro Arg Arg Ser Pro Leu Gly
   435 440 445
   Gln Lys Arg Gly Met Leu Ser Ser Ser Thr Ser Gly Ala Ile Ser Asp
   450 455 460
   Lys Gly Val Leu Arg Pro Gln Lys Glu Ala Val Ser Ser Ser His Gly
   465 470 475 480
   Pro Ser Asp Pro Thr Asp Arg Ala Glu Val Glu Lys Asp Ser Gly His
   485 490 495
   Gly Ser Thr Ser Val Asp Ser Glu Gly Phe Ser Ile Pro Asp Thr Gly
   500 505 510
   Ser His Cys Ser Ser Glu Tyr Ala Ala Ser Ser Pro Gly Asp Arg Gly
   515 520 525
   Ser Gln Glu His Val Asp Ser Gln Glu Lys Ala Pro Glu Thr Asp Asp
   530 535 540
   Ser Phe Ser Asp Val Asp Cys His Ser Asn Gln Glu Asp Thr Gly Cys
   545 550 555 560
   Lys Phe Arg Val Leu Pro Gln Pro Thr Asn Leu Ala Thr Pro Asn Thr
                   565                     570                     575
   Lys Arg Phe Lys Lys Glu Glu Ile Leu Ser Ser Ser Asp Ile Cys Gln
               580                     585                     590
   Lys Leu Val Asn Thr Gln Asp Met Ser Ala Ser Gln Val Asp Val Ala
           595                     600                     605
   Val Lys Ile Asn Lys Lys Val Val Pro Leu Asp Phe Ser Met Ser Ser
       610                     615                     620
   Leu Ala Lys Arg Ile Lys Gln Leu His His Glu Ala Gln Gln Ser Glu
   625                     630                     635                     640
   Gly Glu Gln Asn Tyr Arg Lys Phe Arg Ala Lys Ile Cys Pro Gly Glu
                   645                     650                     655
   Asn Gln Ala Ala Glu Asp Glu Leu Arg Lys Glu Ile Ser Lys Thr Met
               660                     665                     670
   Phe Ala Glu Met Glu Ile Ile Gly Gln Phe Asn Leu Gly Phe Ile Ile
           675                     680                     685
   Thr Lys Leu Asn Glu Asp Ile Phe Ile Val Asp Gln His Ala Thr Asp
       690                     695                     700
   Glu Lys Tyr Asn Phe Glu Met Leu Gln Gln His Thr Val Leu Gln Gly
   705                     710                     715                     720
   Gln Arg Leu Ile Ala Pro Gln Thr Leu Asn Leu Thr Ala Val Asn Glu
                   725                     730                     735
   Ala Val Leu Ile Glu Asn Leu Glu Ile Phe Arg Lys Asn Gly Phe Asp
               740                     745                     750
   Phe Val Ile Asp Glu Asn Ala Pro Val Thr Glu Arg Ala Lys Leu Ile
           755                     760                     765
   Ser Leu Pro Thr Ser Lys Asn Trp Thr Phe Gly Pro Gln Asp Val Asp
       770                     775                     780
   Glu Leu Ile Phe Met Leu Ser Asp Ser Pro Gly Val Met Cys Arg Pro
   785                     790                     795                     800
   Ser Arg Val Lys Gln Met Phe Ala Ser Arg Ala Cys Arg Lys Ser Val
                   805                     810                     815
   Met Ile Gly Thr Ala Leu Asn Thr Ser Glu Met Lys Lys Leu Ile Thr
               820                 825                 830
   His Met Gly Glu Met Asp His Pro Trp Asn Cys Pro His Gly Arg Pro
           835                 840                 845
   Thr Met Arg His Ile Ala Asn Leu Gly Val Ile Ser Gln Asn
       850                 855                 860
<210> 10
   <211> 2771
   <212> DNA
   <213> Homo sapiens
<400> 10
   cgaggcggat cgggtgttgc atccatggag cgagctgaga gctcgagtac agaacctgct 60
   aaggccatca aacctattga tcggaagtca gtccatcaga tttgctctgg gcaggtggta 120
   ctgagtctaa gcactgcggt aaaggagtta gtagaaaaca gtctggatgc tggtgccact 180
   aatattgatc taaagcttaa ggactatgga gtggatctta ttgaagtttc agacaatgga 240
   tgtggggtag aagaagaaaa cttcgaaggc ttaactctga aacatcacac atctaagatt 300
   caagagtttg ccgacctaac tcaggttgaa acttttggct ttcgggggga agctctgagc 360
   tcactttgtg cactgagcga tgtcaccatt tctacctgcc acgcatcggc gaaggttgga 420
   actcgactga tgtttgatca caatgggaaa attatccaga aaacccccta cccccgcccc 480
   agagggacca cagtcagcgt gcagcagtta ttttccacac tacctgtgcg ccataaggaa 540
   tttcaaagga atattaagaa ggagtatgcc aaaatggtcc aggtcttaca tgcatactgt 600
   atcatttcag caggcatccg tgtaagttgc accaatcagc ttggacaagg aaaacgacag 660
   cctgtggtat gcacaggtgg aagccccagc ataaaggaaa atatcggctc tgtgtttggg 720
   cagaagcagt tgcaaagcct cattcctttt gttcagctgc cccctagtga ctccgtgtgt 780
   gaagagtacg gtttgagctg ttcggatgct ctgcataatc ttttttacat ctcaggtttc 840
   atttcacaat gcacgcatgg agttggaagg agttcaacag acagacagtt tttctttatc 900
   aaccggcggc cttgtgaccc agcaaaggtc tgcagactcg tgaatgaggt ctaccacatg 960
   tataatcgac accagtatcc atttgttgtt cttaacattt ctgttgattc agaatgcgtt 1020
   gatatcaatg ttactccaga taaaaggcaa attttgctac aagaggaaaa gcttttgttg 1080
   gcagttttaa agacctcttt gataggaatg tttgatagtg atgtcaacaa gctaaatgtc 1140
   agtcagcagc cactgctgga tgttgaaggt aacttaataa aaatgcatgc agcggatttg 1200
   gaaaagccca tggtagaaaa gcaggatcaa tccccttcat taaggactgg agaagaaaaa 1260
   aaagacgtgt ccatttccag actgcgagag gccttttctc ttcgtcacac aacagagaac 1320
   aagcctcaca gcccaaagac tccagaacca agaaggagcc ctctaggaca gaaaaggggt 1380
   atgctgtctt ctagcacttc aggtgccatc tctgacaaag gcgtcctgag acctcagaaa 1440
   gaggcagtga gttccagtca cggacccagt gaccctacgg acagagcgga ggtggagaag 1500
   gactcggggc acggcagcac ttccgtggat tctgaggggt tcagcatccc agacacgggc 1560
   agtcactgca gcagcgagta tgcggccagc tccccagggg acaggggctc gcaggaacat 1620
   gtggactctc aggagaaagc gcctgaaact gacgactctt tttcagatgt ggactgccat 1680
   tcaaaccagg aagataccgg atgtaaattt cgagttttgc ctcagccaac taatctcgca 1740
   accccaaaca caaagcgttt taaaaaagaa gaaattcttt ccagttctga catttgtcaa 1800
   aagttagtaa atactcagga catgtcagcc tctcaggttg atgtagctgt gaaaattaat 1860
   aagaaagttg tgcccctgga cttttctatg agttctttag ctaaacgaat aaagcagtta 1920
   catcatgaag cacagcaaag tgaaggggaa cagaattaca ggaagtttag ggcaaagatt 1980
   tgtcctggag aaaatcaagc agccgaagat gaactaagaa aagagataag taaaacgatg 2040
   tttgcagaaa tggaaatcat tggtcagttt aacctgggat ttataataac caaactgaat 2100
   gaggatatct tcatagtgga ccagcatgcc acggacgaga agtataactt cgagatgctg 2160
   cagcagcaca ccgtgctcca ggggcagagg ctcatagcac ctcagactct caacttaact 2220
   gctgttaatg aagctgttct gatagaaaat ctggaaatat ttagaaagaa tggctttgat 2280
   tttgttatcg atgaaaatgc tccagtcact gaaagggcta aactgatttc cttgccaact 2340
   agtaaaaact ggaccttcgg accccaggac gtcgatgaac tgatcttcat gctgagcgac 2400
   agccctgggg tcatgtgccg gccttcccga gtcaagcaga tgtttgcctc cagagcctgc 2460
   cggaagtcgg tgatgattgg gactgctctt aacacaagcg agatgaagaa actgatcacc 2520
   cacatggggg agatggacca cccctggaac tgtccccatg gaaggccaac catgagacac 2580
   atcgccaacc tgggtgtcat ttctcagaac tgaccgtagt cactgtatgg aataattggt 2640
   tttatcgcag atttttatgt tttgaaagac agagtcttca ctaacctttt ttgttttaaa 2700
   atgaaacctg ctacttaaaa aaaatacaca tcacacccat ttaaaagtga tcttgagaac 2760
   cttttcaaac c                                                      2771
<210> 11
   <211> 932
   <212> PRT
   <213> Homo sapiens
<400> 11
<210> 12
   <211> 3063
   <212> DNA
   <213> Homo sapiens
<400> 12
   ggcacgagtg gctgcttgcg gctagtggat ggtaattgcc tgcctcgcgc tagcagcaag 60
   ctgctctgtt aaaagcgaaa atgaaacaat tgcctgcggc aacagttcga ctcctttcaa 120
   gttctcagat catcacttcg gtggtcagtg ttgtaaaaga gcttattgaa aactccttgg 180
   atgctggtgc cacaagcgta gatgttaaac tggagaacta tggatttgat aaaattgagg 240
   tgcgagataa cggggagggt atcaaggctg ttgatgcacc tgtaatggca atgaagtact 300
   acacctcaaa aataaatagt catgaagatc ttgaaaattt gacaacttac ggttttcgtg 360
   gagaagcctt ggggtcaatt tgttgtatag ctgaggtttt aattacaaca agaacggctg 420
   ctgataattt tagcacccag tatgttttag atggcagtgg ccacatactt tctcagaaac 480
   cttcacatct tggtcaaggt acaactgtaa ctgctttaag attatttaag aatctacctg 540
   taagaaagca gttttactca actgcaaaaa aatgtaaaga tgaaataaaa aagatccaag 600
   atctcctcat gagctttggt atccttaaac ctgacttaag gattgtcttt gtacataaca 660
   aggcagttat ttggcagaaa agcagagtat cagatcacaa gatggctctc atgtcagttc 720
   tggggactgc tgttatgaac aatatggaat cctttcagta ccactctgaa gaatctcaga 780
   tttatctcag tggatttctt ccaaagtgtg atgcagacca ctctttcact agtctttcaa 840
   caccagaaag aagtttcatc ttcataaaca gtcgaccagt acatcaaaaa gatatcttaa 900
   agttaatccg acatcattac aatctgaaat gcctaaagga atctactcgt ttgtatcctg 960
   ttttctttct gaaaatcgat gttcctacag ctgatgttga tgtaaattta acaccagata 1020
   aaagccaagt attattacaa aataaggaat ctgttttaat tgctcttgaa aatctgatga 1080
   cgacttgtta tggaccatta cctagtacaa attcttatga aaataataaa acagatgttt 1140
   ccgcagctga catcgttctt agtaaaacag cagaaacaga tgtgcttttt aataaagtgg 1200
   aatcatctgg aaagaattat tcaaatgttg atacttcagt cattccattc caaaatgata 1260
   tgcataatga tgaatctgga aaaaacactg atgattgttt aaatcaccag ataagtattg 1320
   gtgactttgg ttatggtcat tgtagtagtg aaatttctaa cattgataaa aacactaaga 1380
   atgcatttca ggacatttca atgagtaatg tatcatggga gaactctcag acggaatata 1440
   gtaaaacttg ttttataagt tccgttaagc acacccagtc agaaaatggc aataaagacc 1500
   atatagatga gagtggggaa aatgaggaag aagcaggtct tgaaaactct tcggaaattt 1560
   ctgcagatga gtggagcagg ggaaatatac ttaaaaattc agtgggagag aatattgaac 1620
   ctgtgaaaat tttagtgcct gaaaaaagtt taccatgtaa agtaagtaat aataattatc 1680
   caatccctga acaaatgaat cttaatgaag attcatgtaa caaaaaatca aatgtaatag 1740
   ataataaatc tggaaaagtt acagcttatg atttacttag caatcgagta atcaagaaac 1800
   ccatgtcagc aagtgctctt tttgttcaag atcatcgtcc tcagtttctc atagaaaatc 1860
   ctaagactag tttagaggat gcaacactac aaattgaaga actgtggaag acattgagtg 1920
   aagaggaaaa actgaaatat gaagagaagg ctactaaaga cttggaacga tacaatagtc 1980
   aaatgaagag agccattgaa caggagtcac aaatgtcact aaaagatggc agaaaaaaga 2040
   taaaacccac cagcgcatgg aatttggccc agaagcacaa gttaaaaacc tcattatcta 2100
   atcaaccaaa acttgatgaa ctccttcagt cccaaattga aaaaagaagg agtcaaaata 2160
   ttaaaatggt acagatcccc ttttctatga aaaacttaaa aataaatttt aagaaacaaa 2220
   acaaagttga cttagaagag aaggatgaac cttgcttgat ccacaatctc aggtttcctg 2280
   atgcatggct aatgacatcc aaaacagagg taatgttatt aaatccatat agagtagaag 2340
   aagccctgct atttaaaaga cttcttgaga atcataaact tcctgcagag ccactggaaa 2400
   agccaattat gttaacagag agtcttttta atggatctca ttatttagac gttttatata 2460
   aaatgacagc agatgaccaa agatacagtg gatcaactta cctgtctgat cctcgtctta 2520
   cagcgaatgg tttcaagata aaattgatac caggagtttc aattactgaa aattacttgg 2580
   aaatagaagg aatggctaat tgtctcccat tctatggagt agcagattta aaagaaattc 2640
   ttaatgctat attaaacaga aatgcaaagg aagtttatga atgtagacct cgcaaagtga 2700
   taagttattt agagggagaa gcagtgcgtc tatccagaca attacccatg tacttatcaa 2760
   aagaggacat ccaagacatt atctacagaa tgaagcacca gtttggaaat gaaattaaag 2820
   agtgtgttca tggtcgccca ttttttcatc atttaaccta tcttccagaa actacatgat 2880
   taaatatgtt taagaagatt agttaccatt gaaattggtt ctgtcataaa acagcatgag 2940
   tctggtttta aattatcttt gtattatgtg tcacatggtt attttttaaa tgaggattca 3000
   ctgacttgtt tttatattga aaaaagttcc acgtattgta gaaaacgtaa ataaactaat 3060
   aac                                                               3063
<210> 13
   <211> 934
   <212> PRT
   <213> Homo sapiens
<400> 13
<210> 14
   <211> 3145
   <212> DNA
   <213> Homo sapiens
<400> 14
   ggcgggaaac agcttagtgg gtgtggggtc gcgcattttc ttcaaccagg aggtgaggag 60
   gtttcgacat ggcggtgcag ccgaaggaga cgctgcagtt ggagagcgcg gccgaggtcg 120
   gcttcgtgcg cttctttcag ggcatgccgg agaagccgac caccacagtg cgccttttcg 180
   accggggcga cttctatacg gcgcacggcg aggacgcgct gctggccgcc cgggaggtgt 240
   tcaagaccca gggggtgatc aagtacatgg ggccggcagg agcaaagaat ctgcagagtg 300
   ttgtgcttag taaaatgaat tttgaatctt ttgtaaaaga tcttcttctg gttcgtcagt 360
   atagagttga agtttataag aatagagctg gaaataaggc atccaaggag aatgattggt 420
   atttggcata taaggcttct cctggcaatc tctctcagtt tgaagacatt ctctttggta 480
   acaatgatat gtcagcttcc attggtgttg tgggtgttaa aatgtccgca gttgatggcc 540
   agagacaggt tggagttggg tatgtggatt ccatacagag gaaactagga ctgtgtgaat 600
   tccctgataa tgatcagttc tccaatcttg aggctctcct catccagatt ggaccaaagg 660
   aatgtgtttt acccggagga gagactgctg gagacatggg gaaactgaga cagataattc 720
   aaagaggagg aattctgatc acagaaagaa aaaaagctga cttttccaca aaagacattt 780
   atcaggacct caaccggttg ttgaaaggca aaaagggaga gcagatgaat agtgctgtat 840
   tgccagaaat ggagaatcag gttgcagttt catcactgtc tgcggtaatc aagtttttag 900
   aactcttatc agatgattcc aactttggac agtttgaact gactactttt gacttcagcc 960
   agtatatgaa attggatatt gcagcagtca gagcccttaa cctttttcag ggttctgttg 1020
   aagataccac tggctctcag tctctggctg ccttgctgaa taagtgtaaa acccctcaag 1080
   gacaaagact tgttaaccag tggattaagc agcctctcat ggataagaac agaatagagg 1140
   agagattgaa tttagtggaa gcttttgtag aagatgcaga attgaggcag actttacaag 1200
   aagatttact tcgtcgattc ccagatctta accgacttgc caagaagttt caaagacaag 1260
   cagcaaactt acaagattgt taccgactct atcagggtat aaatcaacta cctaatgtta 1320
   tacaggctct ggaaaaacat gaaggaaaac accagaaatt attgttggca gtttttgtga 1380
   ctcctcttac tgatcttcgt tctgacttct ccaagtttca ggaaatgata gaaacaactt 1440
   tagatatgga tcaggtggaa aaccatgaat tccttgtaaa accttcattt gatcctaatc 1500
   tcagtgaatt aagagaaata atgaatgact tggaaaagaa gatgcagtca acattaataa 1560
   gtgcagccag agatcttggc ttggaccctg gcaaacagat taaactggat tccagtgcac 1620
   agtttggata ttactttcgt gtaacctgta aggaagaaaa agtccttcgt aacaataaaa 1680
   actttagtac tgtagatatc cagaagaatg gtgttaaatt taccaacagc aaattgactt 1740
   ctttaaatga agagtatacc aaaaataaaa cagaatatga agaagcccag gatgccattg 1800
   ttaaagaaat tgtcaatatt tcttcaggct atgtagaacc aatgcagaca ctcaatgatg 1860
   tgttagctca gctagatgct gttgtcagct ttgctcacgt gtcaaatgga gcacctgttc 1920
   catatgtacg accagccatt ttggagaaag gacaaggaag aattatatta aaagcatcca 1980
   ggcatgcttg tgttgaagtt caagatgaaa ttgcatttat tcctaatgac gtatactttg 2040
   aaaaagataa acagatgttc cacatcatta ctggccccaa tatgggaggt aaatcaacat 2100
   atattcgaca aactggggtg atagtactca tggcccaaat tgggtgtttt gtgccatgtg 2160
   agtcagcaga agtgtccatt gtggactgca tcttagcccg agtaggggct ggtgacagtc 2220
   aattgaaagg agtctccacg ttcatggctg aaatgttgga aactgcttct atcctcaggt 2280
   ctgcaaccaa agattcatta ataatcatag atgaattggg aagaggaact tctacctacg 2340
   atggatttgg gttagcatgg gctatatcag aatacattgc aacaaagatt ggtgcttttt 2400
   gcatgtttgc aacccatttt catgaactta ctgccttggc caatcagata ccaactgtta 2460
   ataatctaca tgtcacagca ctcaccactg aagagacctt aactatgctt tatcaggtga 2520
   agaaaggtgt ctgtgatcaa agttttggga ttcatgttgc agagcttgct aatttcccta 2580
   agcatgtaat agagtgtgct aaacagaaag ccctggaact tgaggagttt cagtatattg 2640
   gagaatcgca aggatatgat atcatggaac cagcagcaaa gaagtgctat ctggaaagag 2700
   agcaaggtga aaaaattatt caggagttcc tgtccaaggt gaaacaaatg ccctttactg 2760
   aaatgtcaga agaaaacatc acaataaagt taaaacagct aaaagctgaa gtaatagcaa 2820
   agaataatag ctttgtaaat gaaatcattt cacgaataaa agttactacg tgaaaaatcc 2880
   cagtaatgga atgaaggtaa tattgataag ctattgtctg taatagtttt atattgtttt 2940
   atattaaccc tttttccata gtgttaactg tcagtgccca tgggctatca acttaataag 3000
   atatttagta atattttact ttgaggacat tttcaaagat ttttattttg aaaaatgaga 3060
   gctgtaactg aggactgttt gcaattgaca taggcaataa taagtgatgt gctgaatttt 3120
   ataaataaaa tcatgtagtt tgtgg                                        3145
<210> 15
   <211> 756
   <212> PRT
   <213> Homo sapiens
<400> 15
<210> 16
   <211> 2484
   <212> DNA
   <213> Homo sapiens
<400> 16